(19) Europäisches Patentamt
European Patent Office
Office européen des brevets

(11) Publication number: **0 398 961 B1**

(12) # EUROPEAN PATENT SPECIFICATION

(45) Date of publication of patent specification: **02.11.94**

(51) Int. Cl.5: **C07K 7/06**, C07K 7/02, A61K 37/43

(21) Application number: **89902190.1**

(22) Date of filing: **18.01.89**

(86) International application number: **PCT/US89/00202**

(87) International publication number: **WO 89/07111 (10.08.89 89/18)**

(54) **POLYPEPTIDE COMPOUNDS HAVING GROWTH HORMONE RELEASING ACTIVITY.**

(30) Priority: **28.01.88 US 149266**

(43) Date of publication of application: **28.11.90 Bulletin 90/48**

(45) Publication of the grant of the patent: **02.11.94 Bulletin 94/44**

(84) Designated Contracting States: **AT BE CH DE FR GB IT LI LU NL SE**

(56) References cited:
**EP-A- 0 018 072**
**WO-A-83/02272**
**WO-A-87/06835**

**Endocrinology, Vol. 114, No. 5, 1984 F.A. Momany et al.: "Conformational Energy Studies and in Vitro and in Vivo Activity Data on Growth Hormone-Releasing Peptides.", page 1531 - page 1536, see the whole article**

(73) Proprietor: **POLYGEN HOLDING CORPORATION**
**1201 North Market Street**
**Wilmington, Delaware 19899 (US)**

(72) Inventor: **BOWERS, Cyril, Yarling**
**484 Audubon Street**
**New Orleans, LA 70118 (US)**
Inventor: **MOMANY, Frank, Alden**
**1-1 Concord Green**
**Concord, MA 02154 (US)**
Inventor: **CHANG, Ching, Hsong**
**16 Robin Drive**
**Fanwood, NJ 07023 (US)**
Inventor: **CODY, Wayne, Livingston, c/o Warner-Lambert Comp.**
**2800 Plymouth Road**
**Ann Arbor, MI 48105 (US)**
Inventor: **HUBBS, John, Clark**
**Route 10, Box 354A**
**Kingsport, TN 37664 (US)**
Inventor: **FOSTER, Charles, Howard**
**1413 Dobyns Drive**
**Kingsport, TN 37664 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 398 961 B1

(74) Representative: **Davies, Jonathan Mark et al**
**Reddie & Grose**
**16 Theobalds Road**
**London WC1X 8PL (GB)**

## Description

This invention relates to novel polypeptide compounds which promote the release of growth hormone when administered to animals. In another aspect, this invention relates to methods for promoting the release and elevation of growth hormone levels in animals by administration of specified growth hormone releasing polypeptide compounds thereto.

### Background of the Invention

It has been established in the scientific literature that the elevation of growth hormone (GH) levels in mammals upon administration of GH-releasing compounds can lead to enhanced body weight and to enhanced milk production if sufficiently elevated GH levels occur upon administration. Further, it is known that the elevation of growth hormone levels in mammals can be accomplished by application of known growth hormone releasing agents, such as the naturally occurring growth hormone releasing hormones.

The elevation of growth hormone levels in mammals can also be accomplished by application of growth hormone releasing peptides, some of which have been previously described, for example, by F. A. Momany in U.S. 4,223,019, U.S. 4,223,020, U.S. 4,223,021, U.S. 4,224,316, U.S. 4,226,857, U.S. 4,228,155, U.S. 4,228,156, U.S. 4,228,157, U.S. 4,228,158, U.S. 4,410,512 and U.S. 4,410,513.

Antibodies to the endogenous growth hormone release inhibitor, somatostatin (SRIF) have also been used to cause elevated GH levels. In this latter example, growth hormone levels are elevated by removing the endogenous GH-release inhibitor (SRIF) before it reaches the pituitary, where it inhabits the release of GH.

Each of these methods for promoting the elevation of growth hormone levels involve materials which are expensive to synthesize and/or isolate in sufficient purity for administration to a target animal. Short chain, relatively simple polypeptides which have the ability to promote the release of growth hormone would be desirable because they should be readily and inexpensively prepared, easily modified chemically and/or physically, as well as easily purified and formulated; and they should have excellent transport properties.

### Objects of the Invention

It is, therefore, an object of the present invention to provide novel growth hormone releasing compounds which are capable of promoting the release and elevation of growth hormone levels in the blood of animals.

It is another object of the present invention to provide methods for promoting the release and/or elevation of growth hormone levels in the blood of animals.

These and other objects of the present invention will become apparent from inspection of the following description and claims.

### Statement of the Invention

In accordance with the present invention, we have discovered several novel polypeptide compounds which promote the release of growth hormone in animals. The preparation, characterization and administration of these novel growth hormone releasing compounds will now be described in greater detail.

### Detailed Description of the Invention

The present invention is based on the discovery of several short chain (i.e., seven up to eleven amino acid residues) polypeptides which promote the release and elevation of growth hormone levels in the blood of animals. The polypeptides contemplated to be within the scope of the present invention are defined by the following generic structure:

AA1-AA2-AA3-Trp-AA5-AA6-AA7-Z,

wherein AA1 is selected from the group consisting of His, 3(NMe)His (i.e., wherein the imidazole ring is methylated at the 3-position), AAO-His and AAO-3(NMe)His; wherein AAO is selected from the group consisting of any naturally occurring L-amino acid, Met(O), DOPA and Abu;

AA2 is selected from the group consisting of DPhe, DTrp, 5-fluoro-D or D/LTrp; 6-fluoro-D or D/LTrp (i.e., wherein the indole ring is fluorinated at the 5- or 6-position), (formyl)DTrp (i.e., DTrp which is formylated at the indole nitrogen), *XTrp, wherein *XTrp is selected from the group consisting of the N-monomethylated DTrp isomers (i.e., ($N^{\alpha}$Me)DTrp and (indole NMe)DTrp), $D^{\alpha}$Nal and $D^{\beta}$Nal;

AA3 is selected from the group consisting of Ala, Gly and Ser;

AA5 is selected from the group consisting of DPhe and (NMe)DPhe;

AA6 is selected from the group consisting of all naturally occurring L-amino acids, dipeptides of the naturally occurring L-amino acids, e.g., Ala-Ala, and compounds of the formula:

$H_2N-(CH_2)_n-CO_2H$,

wherein n = 2-12;

AA7 is selected from the group consisting of Arg, iLys, Lys and Orn;

Z represents the C terminal end group of said polypeptide or the C terminal amino acid(s) plus end group, wherein Z is selected from the group consisting of $-CONH_2$, -COOH, -COOR, -CONHR, $-CONR_2$, $-CH_2OH$ and $-CH_2OR$, wherein R is an alkyl group having 1-6 carbon atoms or an aromatic ring having up to 12 carbon atoms; and wherein Z is alternatively selected from the group consisting of -Gly-Z', -Met-Z', -Lys-Z', -Cys-Z', -Gly-Tyr-Z', and -Ala-Tyr-Z', wherein Z' is selected from the group consisting of $-CONH_2$, -CONHR, -COOH, -COOR, $-CONR_2$, $-CH_2OH$, and $-CH_2OR$, wherein R is as defined above;

and organic or inorganic addition salts of any of said polypeptides;

wherein the amino acid residue abbreviations used are in accordance with the standard peptide nomenclature:

| | |
|---|---|
| Gly | = Glycine |
| Tyr | = L-Tyrosine |
| Ile | = L-Isoleucine |
| Glu | = L-Glutamic Acid |
| Thr | = L-Threonine |
| Phe | = L-Phenylalanine |
| Ala | = L-Alanine |
| Lys | = L-Lysine |
| Asp | = L-Aspartic Acid |
| Cys | = L-Cysteine |
| Arg | = L-Arginine |
| Gln | = L-Glutamine |
| Pro | = L-Proline |
| Leu | = L-Leucine |
| Met | = L-Methionine |
| Ser | = L-Serine |
| Asn | = L-Asparagine |
| His | = L-Histidine |
| Trp | = L-Tryptophan |
| Val | = L-Valine |
| DOPA | = 3,4-Dihydroxyphenylalanine |
| Met(O) | = Methionine Sulfoxide |
| Abu | = $\alpha$-Aminobutyric Acid |
| iLys | = $N^{\epsilon}$-Isopropyl-L-Lysine |
| 4-Abu | = 4-Aminobutyric Acid |
| Orn | = L-Ornithine |
| $D^{\alpha}Nal$ | = $\alpha$-Naphthyl-D-Alanine |
| $D^{\beta}Nal$ | = $\beta$-Naphthyl-D-Alanine |

All three letter amino acid abbreviations preceded by a "D" indicate the D-configuration of the amino acid residue, and abbreviations preceded by a "D/L" indicate a mixture of the D- and L-configurations of the designated amino acid. For purposes of this disclosure, glycine is considered to be included in the term "naturally occurring L-amino acids".

The flexibility associated with the choice of basic, neutral or acidic amino acid residues for amino acids AAO, AA1, AA2, AA3, AA5, AA6 and AA7 provides one with a great deal of control over the physiochemical properties of the desired peptide. Such flexibility provides important advantages for the formulation and delivery of the desired peptide to any given species. Additional flexibility can be imparted by the fact that the moieties R, Z and Z' can be varied as well, thereby providing added control over the physiochemical properties of the desired compound.

Preferred growth hormone releasing compound employed in the practice of the present invention are selected from the group consisting of:

AA1-AA2-AA3-Trp-AA5-Ala-AA7-$NH_2$;

AA1-AA2-AA3-Trp-AA5-Ala-Ala-AA7-$NH_2$;

and

organic or inorganic addition salts of any of said polypeptides; any of which can optionally be preceded by AA0; where AA0, AA1, AA2, AA3, AA5 and AA7 are as defined above.

These compounds are preferred because of their ease of synthesis, proven efficacy at promoting an increase in serum growth hormone levels, and their consequent appeal for commercial scale production and utilization. In addition, these compounds may be advantageous in having physiochemical properties which are desirable for the efficient delivery of such polypeptide compounds to a variety of animal species. Because of the flexibility made possible by the various substitutions at numerous positions of the invention polypeptide compounds, a wide range of delivery vehicles can be employed, by selecting the polar, neutral or non-polar nature of the N-terminal, C-terminal and center portions of these polypeptide compounds so as to be compatible with the desired method of delivery.

In a most preferred embodiment, growth hormone releasing peptides employed in the practice of the present invention are selected from the group consisting of:

His-DTrp-Ala-Trp-DPhe-Ala-Lys-$NH_2$;

His-DTrp-Ala-Trp-DPhe-Ala-Ala-Lys-$NH_2$; and

organic or inorganic addition salts of either of said polypeptides; with the compound:

His-DTrp-Ala-Trp-DPhe-Ala-Lys-$NH_2$, and organic or inorganic addition salts thereof being the presently most preferred growth hormone releasing peptide.

These compounds are the presently most preferred because these shorter chain polypeptides are less expensive to synthesize, and these specific compounds have been shown to have a high level of potency at promoting the increase in serum growth hormone levels.

The compounds of this invention may be used to enhance blood GH levels in animals; enhance milk production in cows; enhance body growth in animals such as mammals (e.g., humans, sheep, bovines, and swine), as well as fish, fowl, other vertebrates and crustaceans; and increase wool and/or fur production in mammals. The amount of body growth is dependent upon the sex and age of the animal species, quantity and identity of the growth hormone releasing compound being administered, route of administration, and the like.

The novel polypeptide compounds of this invention can be synthesized according to the usual methods of solution and solid phase peptide chemistry, or by classical methods known in the art. The solid-phase synthesis is commenced from the C-terminal end of the peptide. A suitable starting material can be prepared, for instance, by attaching the required protected alpha-amino acid to a chloromethylated resin, a hydroxymethyl resin, a benzhydrylamine (BHA) resin, or a para-methyl-benzylhydrylamine (p-Me-BHA) resin. One such chloromethyl resin is sold under the tradename BIOBEADS SX-1 by Bio Rad Laboratories, Richmond, Calif. The preparation of the hydroxymethyl resin is described by Bodansky et al., Chem. Ind. - (London) 38, 1597 (1966). The BHA resin has been described by Pietta and Marshall, Chem. Comm., 650 (1970) and is commercially available from Peninsula Laboratories, Inc., Belmont, California.

After the initial attachment, the alpha-amino protecting group can be removed by a choice of acidic reagents, including trifluoroacetic acid (TFA) or hydrochloric acid (HCl) solutions in organic solvents at room temperature. After removal of the alpha-amino protecting group, the remaining protected amino acids can be coupled stepwise in the desired order. Each protected amino acid can be generally reacted in about a 3-fold excess using an appropriate carboxyl group activator such as dicyclohexylcarbodiimide (DCC) or diisopropyl carbodiimide (DIC) in solution, for example, in methylene chloride ($CH_2Cl_2$) or dimethylformamide (DMF) and mixtures thereof.

After the desired amino acid sequence has been completed, the desired peptide can be cleaved from the resin support by treatment with a reagent such as hydrogen fluoride (HF) which not only cleaves the peptide from the resin, but also cleaves most commonly used side-chain protecting groups. When a chloromethyl resin or hydroxymethyl resin is used, HF treatment results in the formation of the free peptide acid. When the BHA or p-Me-BHA resin is used, HF treatment results directly in free peptide amides.

The solid-phase procedure discussed above is well known in the art and has been described by Stewart and Young, Solid Phase Peptide Synthesis: Second Edn. (Pierce Chemical Co., Rockford, IL, 1984).

Some of the well known solution methods which can be employed to synthesize the peptide moieties of the instant invention are set forth in Bodansky et al., Peptide Synthesis, 2nd Edition, John Wiley & Sons, New York, N.Y. 1976.

In accordance with another embodiment of the present invention, a method is provided for promoting release and/or elevation of growth hormone levels in the blood of an animal. Said method comprises administering to an animal an effective dose of at least one of the above-described polypeptides.

The compounds of this invention can be administered by oral, parenteral (intramuscular (i.m.), intraperitoneal (i.p.), intravenous (i.v.) or subcutaneous (s.c.) injection), nasal, vaginal, rectal or sublingual routes of administration and can be formulated in dose forms appropriate for each route of administration.

Solid dose forms for oral administration include capsules, tablets, pills, powders and granules. In such solid dose forms, the active compound is mixed with at least one inert carrier such as sucrose, lactose, or starch. Such dose forms can also comprise, as is normal practice, additional substances other than inert diluents, e.g., lubricating agents such as magnesium stearate. In the case of capsules, tablets and pills, the dose forms may also comprise buffering agents. Tablets and pills can additionally be prepared with enteric coatings.

Liquid dose forms for oral administration include emulsions, solutions, suspensions, syrups, the elixirs containing inert diluents commonly used in the art, such as water. Besides, such inert diluents, compositions can also include adjuvants, such as wetting agents, emulsifying and suspending agents, and sweetening, flavoring, and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dose forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in a medium of sterile water, or some other sterile injectable medium immediately before use.

As suggested in our copending applications Serial No. 861,968 and Serial No. 37,275, incorporated by reference herein, the novel compounds of the present invention are also useful when administered in combination with growth hormone releasing hormone (i.e., naturally occurring growth hormone releasing hormone, analogs and functional equivalents thereof), as well as in combination with other compounds which promote the release of growth hormone, e.g., growth hormone releasing peptides. Such combinations represent an especially preferred means to administer the growth hormone releasing peptides of the present invention because the combination promotes the release of much more growth hormone than is predicted by the summation of the individual responses for each component of the combination, i.e., the combination provides a synergistic response relative to the individual component. For further detail on the administration of combinations of growth hormone releasing peptides, those of skill in the art are referred to the above-cited applications.

The amount of polypeptide or combination of polypeptides of the present invention administered will vary depending on numerous factors, e.g., the particular animal treated, its age and sex, the desired therapeutic affect, the route of administration and which polypeptide or combination of polypeptides are employed. In all instances, however, a dose effective to promote release and elevation of growth hormone level in the blood of the recipient animal is used. Ordinarily, this dose level falls in the range of between about 0.1 $\mu$g up to 10 mg of total polypeptide per kg of body weight. In general, the administration of combinations of growth hormone releasing peptides will allow for lower doses of the individual growth hormone releasing compounds to be employed relative to the dose levels required for individual growth hormone releasing compounds in order to obtain a similar response, due to the synergistic effect of the combination.

Also included within the scope of the present invention are compositions comprising, as an active ingredient, the organic and inorganic addition salts of the above described polypeptides and combinations thereof; optionally, in association with a carrier, diluent, slow release matrix, or coating.

The organic or inorganic addition salts of the growth hormone releasing compounds and combinations thereof contemplated to be within the scope of the present invention include salts of such organic moieties as acetate, trifluoroacetate, oxalate, valerate, oleate, laurate, benzoate, lactate, tosylate, citrate, maleate, fumarate, succinate, tartrate, naphthalate, and the like; and such inorganic moieties as Group I (i.e., alkali metal salts), Group II (i.e., alkaline earth metal salts) ammonium and protamine salts, zinc, iron, and the like with counterions such as the chloride, bromide, sulfate, phosphate and the like, as well as the organic moieties referred to above.

Pharmaceutically acceptable salts are preferred when administration to human subjects is contemplated. Such salts include the non-toxic alkali metal, alkaline earth metal and ammonium salts commonly used in the pharmaceutical industry including the sodium, potassium, lithium, calcium, magnesium, barium, ammonium and protamine salts which are prepared by methods well known in the art. The term also includes non-toxic acid addition salts which are generally prepared by reacting the compounds of this invention with a suitable organic or inorganic acid. Representative salts include the hydrochloride,

hydrobromide, sulfate, bisulfate, acetate, oxalate, valerate, oleate, laurate, borate, benzoate, lactate, phosphate, tosylate, citrate, maleate, fumarate, succinate, tartrate, napsylate, and the like.

The invention will now be described in greater detail by reference to the following non-limiting examples.

EXAMPLE 1 - Synthesis of the Growth Hormone Releasing Peptides

Paramethyl benzhydrylamine hydrochloride (pMe-BHA•HCl) resin is placed in a reaction vessel on a commercially available automated peptide synthesizer. The resin is substituted with free amine up to a loading of about 5 mmoles per gram. The compounds are prepared by coupling individual amino acids starting at the carboxy terminus of the peptide sequence using an appropriate activing agent, such as N,N'-dicyclohexylcarbodiimide (DCC). The alpha amine of individual amino acids are protected, for example, as the t-butyloxycarbonyl derivative (t-Boc) and the reactive side chain functionalities are protected as outlined in Table 1.

Table 1

| Side Chain Protecting Groups Suitable for Solid Phase Peptide Synthesis | |
|---|---|
| Arginine: | $N^g$-Tosyl |
| Aspartic Acid: | O-Benzyl |
| Cysteine: | S-para-Methylbenzyl |
| Glutamic Acid: | O-Benzyl |
| Histidine: | $N^{im}$-Tosyl |
| Lysine: | $N^\epsilon$-2,4-Dichlorobenzyloxycarbonyl |
| Methionine: | S-Sulfoxide |
| Serine: | O-Benzyl |
| Threonine: | O-Benzyl |
| Tryptophan: | $N^{in}$-Formyl |
| Tyrosine: | 0-2,6-Dichlorobenzyl |

Prior to incorporation of the initial amino acid, the resin is agitated three times (about one minute each) with dichloromethane ($CH_2Cl_2$; about 10 mL/gm of resin), neutralized with three agitations (about two minutes each) of N,N-diisopropylethylamine (DIEA) in dichloromethane (10:90; about 10 mL/gm of resin) and agitated three times (about one minute each) with dichloromethane (about 10 mL/gm of resin). The initial and each of the subsequent amino acids are coupled to the resin using a preformed symmetrical anhydride using about 3.0 times the total amount of the binding capacity of the resin of a suitably protected amino acid and about 1.5 times the total amount of the binding capacity of the resin of DCC in an appropriate amount of dichloromethane. For amino acids with a low dichloromethane solubility, N,N-dimethylformamide (DMF) is added to achieve a homogenous solution. Generally, the symmetrical anhydride is prepared up to 30 minutes prior to introduction into the reaction vessel at room temperature or below. The dicyclohexylurea that forms upon preparation of the symmetrical anhydride is removed via gravity filtration of the solution into the reaction vessel. Progress of the coupling of the amino acid to the resin is commonly monitored via a color test using a reagent such as ninhydrin (which reacts with primary and secondary amines. Upon complete coupling of the protected amino acid to the resin (>99%), the alpha amine protecting group is removed by treatment with acidic reagent(s). A commonly used reagent consists of a solution of trifluoroacetic acid (TFA), and anisole in dichloromethane (45:2:53). The complete procedure for incorporation of each individual amino acid residue onto the resin is outlined in Table 2.

TABLE 2

| Procedure for Incorporation of Individual Amino Acids onto a Resin | | |
|---|---|---|
| Reagent | Agitations | Time/Agitation |
| 1. Dichloromethane | 3 | 1 min. |
| 2. TFA, Anisole, Dichloromethane (45:2:53) | 1 | 2 min. |
| 3. TFA, Anisole, Dichloromethane (45:2:53) | 1 | 20 min. |
| 4. Dichloromethane | 3 | 1 min. |
| 5. DIEA, Dichloromethane (10:90) | 3 | 2 min. |
| 6. Dichloromethane | 3 | 1 min. |
| 7. Preformed symmetrical anhydride | 1 | 15-120 min.* |
| 8. Dichloromethane | 3 | 1 min. |
| 9. iso-Propanol | 3 | 1 min. |
| 10. Dichloromethane | 3 | 1 min. |
| 11. Monitor progress of the coupling reaction** | | |
| 12. Repeat Steps 1-12 for each individual amino acid | | |

*Coupling time depends upon the individual amino acid.
**The extent of coupling can be generally monitored by a color test. If the coupling is incomplete, the same amino acid can be recoupled by repeating Steps 7-11. If the coupling is complete the next amino acid can be coupled.

By employing this method of peptide synthesis, novel resin-bound polypeptides such as:
AA1-AA2-AA3-Trp-AA5-AA6-AA7-Ⓡ
are obtained (wherein AA1, AA2, AA3, AA5, AA6 and AA7 are as defined above, and Ⓡ is a polymeric resin and the functional groups of the constituent amino acids are protected with suitable protecting groups as needed). Specific sequences (in appropriately protected form) which have been prepared include:
His-DTrp-Ala-Trp-DPhe-Ala-Lys-Ⓡ,
His-DTrp-Ala-Trp-DPhe-Ala-Ala-Lys-Ⓡ,
DTrp-Ala-Trp-DPhe-Ala-Ala-Lys-Ⓡ,
DTrp-Ala-Trp-DPhe-Ala-Lys-Ⓡ,
Ala-Trp-DPhe-Ala-Ala-Lys-Ⓡ,
Ala-Trp-DPhe-Ala-Lys-Ⓡ,
Trp-DPhe-Ala-Ala-Lys-Ⓡ,
Trp-DPhe-Ala-Lys-Ⓡ,
DPhe-Ala-Ala-Lys-Ⓡ, and
DPhe-Ala-Lys-Ⓡ.

EXAMPLE 2 - In Vivo GH Release in Rats

Immature female Sprague-Dawley rats were obtained from the Charles River Laboratories (Wilmington, MA). After arrival they were housed at 25°C with a 14:10 hr light:dark cycle. Water and Purina rat chow were available ad libitum. Pups were kept with their mothers until 21 days of age.

Twenty-six day old rats, six rats per treatment group, were anesthetized interperitoneally with 50 mg/Kg of pentobarbital 20 minutes prior to i.v. treatment with peptide. Normal saline with 0.1% gelatin was the vehicle for intravenous (i.v.) injections of the peptides. The anesthetized rats, weighing 55-65 grams, were injected i.v. with the quantity of growth hormone releasing compounds indicated in Table 3. Injection was made as a 0.1 mL solution into the jugular vein.

All animals were sacrificed by guillotine 10 minutes after the final test injection (see Table 3). Trunk blood for the determination of blood GH levels was collected following decapitation. After allowing the blood to clot, it was centrifuged and the serum was separated from the clot. Serum was kept frozen until the day of sampling for radioimmunoassay (RIA) determination of growth hormone levels according to the following procedure, as developed by the National Institute of Arthritis, Diabetes and Digestive and Kidney Diseases (NIADDK).

Reagents are generally added to the RIA analysis tubes at a single sitting, at refrigerator temperature (about 4°C) in the following sequence:

(a) buffer,

(b) "cold" (i.e., non-radioactive) standard or unknown serum sample to be analyzed,
(c) radio-iodinated growth hormone antigen, and
(d) growth hormone antiserum.

Reagent addition is generally carried out so that there is achieved a final RIA tube dilution of about 1:30,000 (antiserum to total liquid volume; vol:vol).

The mixed reagents are then typically incubated at room temperature (about 25 °C) for about 24 hours prior to addition of a second antibody (e.g., goat or rabbit anti-monkey gamma globulin serum) which binds to and causes precipitation of the complexed growth hormone antiserum. Precipitated contents of the RIA tubes are then analyzed for the number of counts in a specified period of time in a gamma scintillation counter. A standard curve is prepared by plotting number of radioactive counts versus growth hormone (GH) level. GH levels of unknowns are then determined by reference to the standard curve.

Serum GH was measured by RIA with reagents provided by the National Hormone and Pituitary Program.

Serum levels in Table 3 are recorded in ng/mL in terms of the rat GH standard of 0.61 International Units/mg (IU/mg). Data is recorded as the mean +/-standard error of the mean (SEM). Statistical analysis was performed with Student's t-test. In Table 3 the results shown are the average of studies with six rats.

Table 3

In Vivo GH Release (ng/mL) Promoted by Growth Hormone Releasing Compounds in Pentobarbital Anesthetized Rats

(Animals Sacrificed 10 Minutes After Final Injection)

| Number | Column A Growth Hormone Releasing Compounds | Total Dose (µg) | Control GH ng/mL | GH Released by Compound in Column A ng/mL |
|---|---|---|---|---|
| 8114* | Ala-His-DTrp-Ala-Trp-DPhe-Lys-$NH_2$ | 0.1 | 287±36 | 497±88 |
| | | 0.3 | 287±36 | 714± 57 |
| | | 1.0 | 287±36 | 1422±321 |
| | | 3.0 | 287±36 | 1616±418 |
| 11009* | Lys-His-DTrp-Ala-Trp-DPhe-Lys-$NH_2$ | 0.1 | 287±36 | 430±89 |
| | | 0.3 | 287±36 | 569±106 |
| | | 1.0 | 287±36 | 1561±252 |
| | | 3.0 | 287±36 | 2303±104 |
| 12676 | His-DTrp-Ala-Trp-DPhe-Ala-Lys-$NH_2$ | 0.3 | 111±25 | 796±177 |
| | | 3.0 | 111±25 | 4565±489 |
| 12676 | His-DTrp-Ala-Trp-DPhe-Ala-Lys-$NH_2$ | 0.1 | 220±29 | 223±74 |
| | | 0.1 | 220±29 | 420±105 |
| | | 0.3 | 220±29 | 653±93 |
| | | 0.3 | 220±29 | 900±163 |
| | | 1.0 | 220±29 | 1825±508 |
| | | 1.0 | 220±29 | 1965±366 |
| | | 3.0 | 220±29 | 4553±670 |
| | | 3.0 | 220±29 | 2820±540 |
| 12676 | His-DTrp-Ala-Trp-DPhe-Ala-Lys-$NH_2$ | 0.1 | 224±47 | 434±60 |
| | | 0.3 | 224±47 | 714±82 |
| | | 1.0 | 224±47 | 1552±210 |
| | | 3.0 | 224±47 | 4005±538 |
| 13522 | His-DTrp-Ala-Trp-DPhe-Ala-Ala-Lys-$NH_2$ | 0.1 | 224±47 | 587±85 |
| | | 0.3 | 224±47 | 1137±167 |
| | | 1.0 | 224±47 | 1978±253 |
| | | 3.0 | 224±47 | 4178±816 |
| 13522 | His-DTrp-Ala-Trp-DPhe-Ala-Ala-Lys-$NH_2$ | 0.1 | 220±29 | 479±99 |
| | | 0.3 | 220±29 | 854±125 |
| | | 1.0 | 220±29 | 2472±696 |
| | | 3.0 | 220±29 | 3627±622 |

*Comparison Peptides

Table 3 (Cont'd.)

In Vivo GH Release (ng/mL) Promoted by Growth Hormone Releasing Compounds in Pentobarbital Anesthetized Rats
(Animals Sacrificed 10 Minutes After Final Injection)

| Number | Column A Growth Hormone Releasing Compounds | Total Dose (µg) | Control GH ng/mL | GH Released by Compound in Column A ng/mL |
|---|---|---|---|---|
| 13154 | His-DTrp-Ala-Trp-DPhe-Ser-Lys-$NH_2$ | 3.0 | 111±25 | 3193±296 |
| 13154 | His-DTrp-Ala-Trp-DPhe-Ser-Lys-$NH_2$ | 0.1 | 165±29 | 128±24 |
| | | 0.3 | 165±29 | 340±58 |
| | | 1.0 | 165±29 | 794±68 |
| | | 3.0 | 165±29 | 1460±375 |
| 13157 | His-DTrp-Ala-Trp-DPhe-Leu-Lys-$NH_2$ | 3.0 | 111±25 | 1644±296 |
| 13157 | His-DTrp-Ala-Trp-DPhe-Leu-Lys-$NH_2$ | 0.1 | 165±29 | 152±20 |
| | | 0.3 | 165±29 | 250±44 |
| | | 1.0 | 165±29 | 442±69 |
| | | 3.0 | 165±29 | 831±293 |
| | | 0.1 | 239±36 | 441±103 |
| | | 0.3 | 239±36 | 647±122 |
| | | 1.0 | 239±36 | 637±183 |
| | | 3.0 | 239±36 | 1661±414 |
| 13119 | His-DTrp-Ala-Trp-DPhe-Asp-Lys-$NH_2$ | 0.3 | 188±14 | 254±59 |
| | | 1.0 | 188±14 | 352±67 |
| | | 3.0 | 188±14 | 1226±477 |

In Table 3, compounds of the invention are shown to promote the release and elevation of growth hormone levels in the blood of rats to which such compounds have been administered.

EXAMPLE 3 - Administration of a Combination of GH-Releasing Compounds

The procedure of Example 2 was repeated, except the rats were not anesthetized nor were they pretreated with pentobarbital, and a combination of peptides were administered to the rats. The compounds administered, the dose level and results are set forth in Table 4.

Table 4
In Vivo Synergistic Effects in Unanesthetized Rats of Invention Compound with Group 1* and/or Group 3* Compounds

| Compound Administered, dose (µg)* | GH Released, mg/mL |
|---|---|
| Control | 9±1 |
| Invention Compound, 3µg | 58±19 |
| Comparison Compound, 3µg | 52±19 |
| Group 1 Compound, 3µg | 327±74 |
| Invention + Group 1 | 1105±123 |
| Comparison + Group 1 | 1480±206 |
| Group 3 Compound, 10µg | 183±55 |
| Invention + Group 3 | 1118±34 |
| Comparison + Group 3 | 2018±369 |
| Invention + Group 1 + Group 3 | 3345±489 |
| Comparison + Group 1 + Group 3 | 4651±232 |

*Group 1 and Group 3 compounds are described in detail in S.N. 861,968 and 37,275 which have been incorporated by reference herein. All compounds employed in these studies have the following sequences:

Invention Compound – His–DTrp–Ala–Trp–DPhe–Ala–Lys–$NH_2$;
Comparison Compound – His–DTrp–Ala–Trp–DPhe–Lys–$NH_2$;
Group 1 Compound – Tyr–Ala–Asp–Ala–Ile–Phe–Thr–Asn–Ser–Tyr–Arg–Lys–Val–Leu–Gly–Gln–Leu–Ser–Ala–Arg–Lys–Leu–Leu–Gln–Asp–Ile–Nle–Ser–Arg–$NH_2$;
Group 3 Compound – Tyr–DArg–Phe–Gly–$NH_2$

The results in Table 4 demonstrate that the invention compound displays a similar synergistic response to that obtained with comparison compound (which has previously been shown to give a synergistic response) when administered in combination with exemplary Group 1 and/or Group 3 compounds.

EXAMPLE 4 - In Vivo Growth Hormone Release Study - Cows

Six multiparous lactating Holstein cows (mean body weight 575 kg) were housed in a dairy barn. The cow diet consisted of a forage to concentrate ratio of 50:50 with 70% of the forage dry matter as corn silage and 30% as alfalfa hay. The concentrate portion of the diet contained corn and soybean meal in adequate quantities to provide a total mixed ration. The ration was balanced following NRC guidelines to meet the nutrient requirements (i.e., dry matter, protein, energy, crude fiber, minerals and vitamins) of dairy cows in early to mid-lactation. Cows were fed twice daily.

Catheters were inserted into the jugular vein for withdrawal of blood samples and iv. injections of peptides. Approximately 4 mL of saline was flushed through the catheter after each blood drawing. Six mL blood samples were collected between about 12:20 PM and 4 PM at -40, -20, -10, 0, +5, +10, +15, +20, +30, +40, +60, +80, +100, +140, and +160 minutes, on each day of the study. Normal saline or peptides dissolved in normal saline was injected i.v. through the catheter at 0 time to the unanesthetized cows. The saline/peptide was infused in bolus (5.0 mL volume). The blood was collected in EDTA treated tubes, centrifuged and the plasma separated from the pellet. Plasma was kept frozen until the day of sampling for radioimmunoassay (RIA) of growth hormone. Plasma GH was measured by RIA with reagents provided by the NIADDK. The GH levels are reported in terms of ng/mL of a bovine GH reference preparation, NIH-GH-B18, which is equivalent to 3.2 IU/mg. Data is recorded as the mean± the standard error of the mean (SEM). Statistical analysis was performed with the Student's t-test. Results are presented in Table 5.

Table 5

Relative Potencies of His-DTrp-Ala-Trp-DPhe-Lys-$NH_2$ (Comparison A), Lys-His-DTrp-Ala-Trp-DPhe-Lys-$NH_2$ (Comparison B) and His-DTrp-Ala-Trp-DPhe-Ala-Lys-$NH_2$ (Invention) In Lactating Dairy Cows

| Compounds | Dose Level | | | |
|---|---|---|---|---|
| | 3 mcg/kg bw | | 9 mcg/kg bw | |
| | GH AUC* ng-min/mL | Log (GH AUC) | GH AUC ng-min/mL | Log (GH AUC) |
| Comparison A | 1,485±1,008 | 6.86±0.38 | 3,734±1,008 | 7.82±0.38 |
| Comparison B | 795±1,008 | 6.72±0.38 | 3,129±1,008 | 7.81±0.38 |
| Invention | 1,592±1,008 | 7.00±0.38 | 6,159±1,008 | 8.46±0.38 |

*GH AUC is GH area under the curve over 180 minutes after bolus IV infusion; all GH values were corrected for differences in molecular weights of each compound.

In Table 5, invention compound is shown to promote The release and elevation of growth hormone levels in the blood of lactating dairy cows to which the compound has been administered. The level of growth hormone release observed is greater than or equal to the levels observed with previously disclosed novel growth hormone releasing peptides.

## Claims

**1.** A polypeptide capable of promoting the release and elevation of growth hormone levels in the blood of a recipient animal, wherein said polypeptide is selected from the group consisting of polypeptides defined by the generic structure:

AA1-AA2-AA3-Trp-AA5-AA6-AA7-Z,

wherein AA1 is selected from the group consisting of His and 3(NMe)His (i.e., wherein the imidazole ring is methylated at the 3-position); AAO-His and AAO-3(NMe)His; wherein AAO is selected from the group consisting of any naturally occurring L-amino acid, Met(O), DOPA and Abu;

AA2 is selected from the group consisting of DPhe, DTrp, 5-fluoro-D or D/LTrp; 6-fluoro-D or D/LTrp (i.e., wherein the indole ring is fluorinated at the 5- or 6-position), (formyl)DTrp (i.e., DTrp which is formylated at the indole nitrogen), *XTrp, wherein *XTrp is selected from the group consisting of the N-monomethylated DTrp isomers (i.e., ($N^\alpha$Me)DTrp and (indole NMe)DTrp), $D^\alpha$Nal and $D^\beta$Nal;

AA3 is selected from the group consisting of Ala, Gly and Ser;

AA5 is selected from the group consisting of DPhe and (NMe)DPhe;
AA6 is selected from the group consisting of all naturally occurring L-amino acids, dipeptides of the naturally occurring L-amino acids, e.g., Ala-Ala, and compounds of the formula:

$H_2N-(CH_2)_n-CO_2H$,

wherein n = 2-12;
AA7 is selected from the group consisting of Arg, iLys, Lys and Orn;
Z represents the C terminal end group of said polypeptide or the C terminal amino acid(s) plus end group, wherein Z is selected from the group consisting of $-CONH_2$, -COOH, -COOR, -CONHR, $-CONR_2$, $-CH_2OH$ and $-CH_2OR$, wherein R is an alkyl group having 1-6 carbon atoms or an aromatic ring having up to 12 carbon atoms; and wherein Z is alternatively selected from the group consisting of -Gly-Z', -Met-Z', -Lys-Z', -Cys-Z', -Gly-Tyr-Z', and -Ala-Tyr-Z', wherein Z' is selected from the group consisting of $-CONH_2$, -COOH, -CONHR, -COOR, $-CONR_2$, $-CH_2OH$, and $-CH_2OR$, wherein R is as defined above;
and organic or inorganic addition salts of any of said polypeptides;
wherein the amino acid residue abbreviations used are in accordance with the standard peptide nomenclature:

| | | |
|---|---|---|
| Gly | = | Glycine |
| Tyr | = | L-Tyrosine |
| Ile | = | L-Isoleucine |
| Glu | = | L-Glutamic Acid |
| Thr | = | L-Threonine |
| Phe | = | L-Phenylalanine |
| Ala | = | L-Alanine |
| Lys | = | L-Lysine |
| Asp | = | L-Aspartic Acid |
| Cys | = | L-Cysteine |
| Arg | = | L-Arginine |
| Gln | = | L-Glutamine |
| Pro | = | L-Proline |
| Leu | = | L-Leucine |
| Met | = | L-Methionine |
| Ser | = | L-Serine |
| Asn | = | L-Asparagine |
| His | = | L-Histidine |
| Trp | = | L-Tryptophan |
| Val | = | L-Valine |
| DOPA | = | 3,4-Dihydroxyphenylalanine |
| Met(O) | = | Methionine Sulfoxide |
| Abu | = | $\alpha$-Aminobutyric Acid |
| iLys | = | $N^{\epsilon}$-Isopropyl-L-Lysine |
| 4-Abu | = | 4-Aminobutyric Acid |
| Orn | = | L-Ornithine |
| $D^{\alpha}$Nal | = | $\alpha$-Naphthyl-D-Alanine |
| $D^{\beta}$Nal | = | $\beta$-Naphthyl-D-Alanine |

All three letter amino acid abbreviations preceded by a "D" indicate the D-configuration of the amino acid residue; abbreviations preceded by a "D/L" indicate a mixture of the D-and L-configurations of the designated amino acids; and glycine is included in the scope of the term "naturally occurring L-amino acids".

**2.** A polypeptide in accordance with Claim 1, wherein said polypeptide is selected from the group consisting of:
AA1-AA2-AA3-Trp-AA5-Ala-AA7-$NH_2$;
AA1-AA2-AA3-Trp-AA5-Ala-Ala-AA7-$NH_2$;
and
organic or inorganic addition salts of any of said polypeptides.

3. A polypeptide in accordance with Claim 1 wherein said polypeptide is selected from the group consisting of:
His-Dtrp-Ala-Trp-DPhe-Ala-Lys-$NH_2$;
His-DTrp-Ala-Trp-DPhe-Ala-Ala-Lys-$NH_2$; and
organic or inorganic addition salts of either of said polypeptides.

4. Method of promoting the release and elevation of blood growth hormone levels in animals by administering thereto an effective amount of at least one of the polypeptides set forth in any one of claims 1, 2, or 3; excluding any method for therapeutic treatment of the human or animal body.

5. A compound of the formula:

AA1-AA2-AA3-Trp-AA5-AA6-AA7-Ⓡ

wherein AA1 is selected from the group consisting of His and 3(NMe)His (i.e., wherein the imidazole ring is methylated at the 3-position); AAO-His, AAO-3(NMe)His; wherein AAO is any naturally occurring L-amino acid;
AA2 is selected from the group consisting of DPhe, DTrp, 5-fluoro-D or D/LTrp; 6-fluoro-D or D/LTrp (i.e., wherein the indole ring is fluorinated at the 5- or 6-position), (formyl)DTrp (i.e., DTrp which is formylated at the indole nitrogen), *XTrp, wherein *XTrp is selected from the group consisting of the N-monomethylated DTrp isomers (i.e., ($N^\alpha$Me)DTrp and (indole NMe)DTrp), $D^\alpha$Nal and $D^\beta$Nal;
AA3 is selected from the group consisting of Ala, Gly and Ser;
AA5 is selected from the group consisting of DPhe and (NMe)DPhe;
AA6 is selected from the group consisting of all naturally occurring L-amino acids, dipeptides of the naturally occurring L-amino acids, e.g., Ala-Ala, and compounds of the formula:

$H_2N(CH_2)_nCO_2H$

wherein n = 2-12;
AA7 is selected from the group consisting of Arg, iLys, Lys and Orn;
and wherein Ⓡ is a polymeric resin and functional groups of the constituent amino acids are protected with suitable protecting groups as needed.

6. A compound of the formula:

His-DTrp-Ala-Trp-DPhe-Ala-Lys-Ⓡ

wherein Ⓡ is a polymeric resin and functional groups of the constituent amino acids are protected with suitable protecting groups as needed.

7. A compound of the formula:

His-DTrp-Ala-Trp-DPhe-Ala-Ala-Lys-Ⓡ

wherein Ⓡ is a polymeric resin and functional groups of the constituent amino acids are protected with suitable protecting groups as needed.

8. A resin-bound polypeptide selected from the group consisting of:
DTrp-Ala-Trp-DPhe-Ala-Ala-Lys-Ⓡ,
DTrp-Ala-Trp-DPhe-Ala-Lys-Ⓡ,
Ala-Trp-Dphe-Ala-Ala-Lys-Ⓡ,
Ala-Trp-DPhe-Ala-Lys-Ⓡ,
Trp-DPhe-Ala-Ala-Lys-Ⓡ,
Trp-DPhe-Ala-Lys-Ⓡ,
DPhe-Ala-Ala-Lys-Ⓡ , and
DPhe-Ala-Lys-Ⓡ.
wherein Ⓡ is a polymeric resin and functional groups of the constituent amino acids are protected with suitable protecting groups as needed.

**9.** A combination effective to cause the release and elevation of the level of growth hormone in the blood of an animal, the combination comprising an effective amount of polypeptides selected from at least two different groups of Group 1 polypeptides, Group 2 polypeptides or Group 3 polypeptides;
wherein Group 1 polypeptides are selected from any of the naturally occurring growth hormone releasing hormones and functional equivalents thereof, wherein said polypeptides act at the growth hormone releasing hormone receptor of mammals and other vertebrates, and crustaceans;
Group 2 polypeptides are selected from any of the polypeptides having the structure:
AA1-AA2-AA3-Trp-AA5-AA6-AA7-Z,
wherein AA1 is selected from the group consisting of His and 3(NMe)His (i.e., wherein the imidazole ring is methylated at the 3-position); AAO-His and AAO-3(NMe)His; wherein AAO is any naturally occurring L-amino acid;
AA2 is selected from the group consisting of DPhe, DTrp, 5-fluoro-D or D/LTrp; 6-fluoro-D or D/LTrp (i.e., wherein the indole ring is fluorinated at the 5- or 6-position), (formyl)DTrp (i.e., DTrp which is formylated at the indole nitrogen), *XTrp, wherein *XTrp is selected from the group consisting of the N-monomethylated DTrp isomers (i.e., ($N^\alpha$Me)DTrp and (indole NMe)DTrp), $D^\alpha$Nal and $D^\beta$Nal;
AA3 is selected from the group consisting of Ala, Gly and Ser;
AA5 is selected from the group consisting of DPhe and (NMe)DPhe;
AA6 is selected from the group consisting of all naturally occurring L-amino acids, dipeptides of the naturally occurring L-amino acids, e.g., Ala-Ala, and compounds of the formula:

$H_2N-(CH_2)_n-CO_2H$,

wherein n = 1-12;
AA7 is selected from the group consisting of Arg, iLys, Lys and Orn;
Z represents the C terminal end group of said polypeptide or the C terminal amino acid(s) plus end group, wherein Z is selected from the group consisting of $-CONH_2$, -COOH, -COOR, -CONHR, $-CONR_2$, $-CH_2OH$ and $-CH_2OR$, wherein R is an alkyl group having 1-6 carbon atoms or an aromatic ring having up to 12 carbon atoms; and wherein Z is alternatively selected from the group consisting of -Gly-Z', -Met-Z', -Lys-Z', -Cys-Z', -Gly-Tyr-Z', and -Ala-Tyr-Z', wherein Z' is selected from the group consisting of $-CONH_2$, -COOH, -CONHR, -COOR, $-CONR_2$, $-CH_2OH$, and $-CH_2OR$, wherein R is as defined above; and organic or inorganic addition salts of any of said polypeptides;
wherein the amino acid residue abbreviations used are in accordance with the standard peptide nomenclature:

| | |
|---|---|
| Gly | = Glycine |
| Tyr | = L-Tyrosine |
| Ile | = L-Isoleucine |
| Glu | = L-Glutamic Acid |
| Thr | = L-Threonine |
| Phe | = L-Phenylalanine |
| Ala | = L-Alanine |
| Lys | = L-Lysine |
| Asp | = L-Aspartic Acid |
| Cys | = L-Cysteine |
| Arg | = L-Arginine |
| Gln | = L-Glutamine |
| Pro | = L-Proline |
| Leu | = L-Leucine |
| Met | = L-Methionine |
| Ser | = L-Serine |
| Asn | = L-Asparagine |
| His | = L-Histidine |
| Trp | = L-Tryptophan |
| Val | = L-Valine |
| DOPA | = 3,4-Dihydroxyphenylalanine |
| Gly-ol | = 2-Aminoethanol |
| Hyp | = trans-4-Hydroxy-L-Proline |
| Met(O) | = Methionine sulfoxide |
| Met(O)-ol | = Methionine sulfoxide alcohol |

Sar = Sarcosine
Sar-ol = Sarcosine alcohol
Thz = L-Thiazolidine-4-carboxylic Acid
iLys = $N^{\epsilon}$-Isopropyl-L-Lysine
4-Abu = 4-Aminobutyric Acid
Orn = L-Ornithine
$D^{\alpha}$Nal = $\alpha$-Naphthyl-D-Alanine
$D^{\beta}$Nal = $\beta$-Naphthyl-D-Alanine

All three letter amino acid abbreviations preceded by a "D" indicate the D-configuration of the amino acid residue; abbreviations preceded by a "D/L" indicate a mixture of the D-and L-configurations of the designated amino acids; and glycine is included in the scope of the term "naturally occurring L-amino acids"; and

Group 3 polypeptides are selected from any of the polypetides having the structure:

Tyr-DArg-Phe-$NH_2$;
Tyr-DAla-Phe-$NH_2$;
Tyr-DArg($NO_2$)-Phe-$NH_2$;
Tyr-DMet(O)-Phe-$NH_2$;
Tyr-DAla-Phe-Gly-$NH_2$;
Tyr-DArg-Phe-Gly-$NH_2$;
Tyr-DThr-Phe-Gly-$NH_2$;
Phe-DArg-Phe-Gly-$NH_2$;
Tyr-DArg-Phe-Sar;
Tyr-DAla-Gly-Phe-$NH_2$;
Tyr-DArg-Gly-Trp-$NH_2$;
Tyr-DArg-($NO_2$)-Phe-Gly-$NH_2$;
Tyr-DMet(O)-Phe-Gly-$NH_2$;
(NMe)Tyr-DArg-Phe-Sar-$NH_2$;
Tyr-DArg-Phe-Gly-ol;
Tyr-DArg-Gly-(NMe)Phe-$NH_2$;
Tyr-DArg-Phe-Sar-ol;
Tyr-DAla-Phe-Sar-ol;
Tyr-DAla-Phe-Gly-Tyr-$NH_2$;
Gly-Tyr-DArg-Phe-Gly-$NH_2$;
Tyr-DThr-Gly-Phe-Thz-$NH_2$;
Gly-Tyr-DAla-Phe-Gly-$NH_2$;
Tyr-DAla-Phe-Gly-ol;
Tyr-DAla-Gly-(NMe)Phe-Gly-ol;
Tyr-DArg-Phe-Sar-$NH_2$;
Tyr-DAla-Phe-Sar-$NH_2$;
Tyr-DAla-Phe-Sar;
Tyr-DAla-Gly-(NMe)Phe-$NH_2$;
Sar-Tyr-DArg-Phe-Sar-$NH_2$;
Tyr-DCys-Phe-Gly-DCys-$NH_2$ (cyclic disulfide);
Tyr-DCys-Phe-Gly-DCys-$NH_2$ (free dithiol);
Tyr-DCys-Gly-Phe-DCys-$NH_2$ (cyclic disulfide);
Tyr-DCys-Gly-Phe-DCys-$NH_2$ (free dithiol);
Tyr-DAla-Phe-Gly-Tyr-Pro-Ser-$NH_2$;
Tyr-DAla-Phe-Sar-Tyr-Pro-Ser-$NH_2$;
Tyr-DAla-Phe-Sar-Phe-Pro-Ser-$NH_2$;
Tyr-DAla-Phe-Gly-Tyr-Hyp-Ser-$NH_2$;
Tyr-DAla-Phe-Sar-Tyr-Hyp-Ser-$NH_2$;
Tyr-DAla-Phe-Sar-Phe-Hyp-Ser-$NH_2$;
Tyr-DArg-Phe-Gly-Tyr-Hyp-Ser-$NH_2$;
Tyr-DArg-Phe-Sar-Tyr-Pro-Ser-$NH_2$;
Tyr-DArg-Phe-Sar-Tyr-Hyp-Ser-$NH_2$;
Tyr-DArg-Phe-Gly-Tyr-Pro-Ser-$NH_2$;
and
organic or inorganic addition salts of any of said polypeptides of Group 3;

wherein said combination is administered in a ratio such that said combination is effective to cause the synergistic release and elevation of growth hormone in the blood of such animal.

**10.** Combination of Claim 9 wherein said Group 1 polypeptides are selected from any of the polypeptides:

(a) having the following amino acid sequences in positions 1-44 (numbered from N terminus to C terminus):

(#144) YADAIFTNSYRKVLGQLSARKLLQDIMSRQQGESNQERGARARL-X,
(#145) YADAIFTNSYRKVLGQLSARKLLQDIMSRQQGERNQEQGARVRL-X,
(#146) YADAIFTNSYRKVLGQLSARKLLQDIMNRQQGERNQEQGAKVRL-X,
(#148) YADAIFTNSYRKILGQLSARKLLQDIMNRQQGERHQEQGAKVRL-X,
(#149) HADAIFTSSYRRILGQLYARKLLHEIMNRQQGERNQEQRSRFN-X; and functional equivalents thereof;

wherein the C-terminal amino acid has the following truncated general formula:

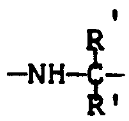

wherein each R' independently represents the substituents of the particular amino acid residue, e.g.; hydrogen, alkyl, aryl, amino or acid substituents; X denotes the C terminal end group and is selected from $-CONH_2$, -COOH, -COOR, -CONRR, $-CH_2OH$, and $-CH_2OR$, where R is an alkyl group having 1-6 carbon atoms or an aromatic ring having up to 12 carbon atoms; and wherein the amino acid residue abbreviations used are in accordance with the standard peptide nomenclature:

G = Gly (Glycine),
Y = Tyr (L-Tyrosine),
I = Ile (L-Isoleucine),
E = Glu (L-Glutamic Acid),
T = Thr (L-Threonine),
F = Phe (L-Phenylalanine),
A = Ala (L-Alanine),
K = Lys (L-Lysine),
D = Asp (L-Aspartic Acid),
C = Cys (L-Cysteine),
R = Arg (L-Arginine),
Q = Gln (L-Glutamine),
P = Pro (L-Proline),
L = Leu (L-Leucine),
M = Met (L-Methionine),
S = Ser (L-Serine),
N = Asn (L-Asparagine),
H = His (L-Histidine),
W = Trp (L-Tryptophan), and
V = Val (L-Valine);
Nle = Norleucine
Sar = Sarcosine
Sar-ol = Sarcosine Alcohol
Gly-ol = 2-Aminoethanol
Met(O) = Methionine Sulfoxide

(b) any one of said (a) polypeptides having the following amino acid substitutions:
Position 1 of (#144-#148) is DTyr or His;
Position 1 of (#149) is Tyr or DHis;
Position 2 of (#144-#149) is (NMe)DAla or Aib or DAla;
Position 3 of (#144-#149) is DAsp;
Position 4 of (#144-#149) is DAla; and
Position 1 + 2 of (#144-#149) is:
$DTyr^1 + DAla^2$, $DTyr^1 + (NMe)DAla^2$, or $DTyr^1 + Aib^2$;

(c) any one of said (a) or (b) polypeptides having a substitution of Nle for Met at Position 27;
(d) any one of said (a), (b) or (c) polypeptides in which the N-terminus $-NH_2$ is replaced by -NHCOR and wherein R is an alkyl group having 1 to 6 carbon atoms, or an aromatic ring having up to 12 carbon atoms;
(e) fragments of any one of said (a), (b), (c) or (d) polypeptides which contain at least the amino acid residues of positions 1-29;
(f) having the following specific amino acid sequences in positions 1-29 (numbered from N terminus to C terminus):
YADAIFTNSYRKVLQQLAARKLLQDIMSR-X,
YADAIFTNSYRKVLQQLLARKLLQDIMSR-X,
YSDAIFSNAYRKILQQLLARKLLQDIMOR-X,
YADAIFSNAYRKILQQLLARKLLQDIMQR-X,
YADAIFSSAYRRLLAQLASRRLLQELLAR-X,
YADAIFTNCYRKVLCQLSARKLLQDIMSR-X (linear dithiol), and
YADAIFTNCYRKVLCQLSARKLLQDIMSR-X (cyclic disulfide);
wherein the C-terminal amino acid and X are as defined above; and modification of any one of these group (f) compounds in accordance with the modifications set forth in (b), (c) and (d) above; and
(g) organic or inorganic addition salts of any of said (a), (b), (c), (d), (e) or (f) polypeptides of Group 1.

**11.** Combination of Claim 9 comprising a compound from each of Group 1 polypeptides and Group 2 polypeptides.

**12.** Combination of Claim 9 comprising a compound from each of Group 2 polypeptides and Group 3 polypeptides.

**13.** Combination of Claim 9 comprising a compound from each of Group 1 polypeptides, Group 2 polypeptides and Group 3 polypeptides.

**14.** Method of causing release and elevation of the level of growth hormone in the blood of an animal; excluding any method for therapeutic treatment of the human or animal body, comprising administering an effective dose of a combination comprising polypeptides selected from at least two different groups of Group 1 polypeptides, Group 2 polypeptides or Group 3 polypeptides;
wherein Group 1 polypeptides are selected from any of the naturally occurring growth hormone releasing hormones and functional equivalents thereof, wherein said polypeptides act at the growth hormone releasing hormone receptor of mammals and other vertebrates, and crustaceans;
Group 2 polypeptides are selected from any of the polypeptides having the structure:
AA1-AA2-AA3-Trp-AA5-AA6-AA7-Z,
wherein AA1 is selected from the group consisting of His and 3(NMe)His (i.e., wherein the imidazole ring is methylated at the 3-position); AAO-His and AAO-3(NMe)His; wherein AAO is any naturally occurring L-amino acid;
AA2 is selected from the group consisting of DPhe, DTrp, 5-fluoro-D or D/LTrp; 6-fluoro-D or D/LTrp (i.e., wherein the indole ring is fluorinated at the 5- or 6-position), (formyl)DTrp (i.e., DTrp which is formylated at the indole nitrogen), *XTrp, wherein *XTrp is selected from the group consisting of the N-monomethylated DTrp isomers (i.e., ($N^\alpha$Me)DTrp and (indole NMe)DTrp), $D^\alpha$Nal and $D^\beta$Nal;
AA3 is selected from the group consisting of Ala, Gly and Ser;
AA5 is selected from the group consisting of DPhe and (NMe)DPhe;
AA6 is selected from the group consisting of all naturally occurring L-amino acids, dipeptides of the naturally occurring L-amino acids, e.g., Ala-Ala, and compounds of the formula:

$$H_2N-(CH_2)_n-CO_2H,$$

wherein n = 2-12;
AA7 is selected from the group consisting of Arg, iLys, Lys and Orn;
Z represents the C terminal end group of said polypeptide or the C terminal amino acid(s) plus end group, wherein Z is selected from the group consisting of $-CONH_2$, -COOH, -COOR, -CONHR, $-CONR_2$, $-CH_2OH$ and $-CH_2OR$, wherein R is an alkyl group having 1-6 carbon atoms or an aromatic ring having up to 12 carbon atoms; and wherein Z is alternatively selected from the group consisting of -Gly-Z',

-Met-Z', -Lys-Z', -Cys-Z', -Gly-Tyr-Z', and -Ala-Tyr-Z', wherein Z' is selected from the group consisting of $-CONH_2$, -COOH, -CONHR, -COOR, $-CONR_2$, $-CH_2OH$, and $-CH_2OR$, wherein R is as defined above; and organic or inorganic addition salts of any of said polypeptides;
wherein the amino acid residue abbreviations used are in accordance with the standard peptide nomenclature:

| | | |
|---|---|---|
| Gly | = | Glycine |
| Tyr | = | L-Tyrosine |
| Ile | = | L-Isoleucine |
| Glu | = | L-Glutamic Acid |
| Thr | = | L-Threonine |
| Phe | = | L-Phenylalanine |
| Ala | = | L-Alanine |
| Lys | = | L-Lysine |
| Asp | = | L-Aspartic Acid |
| Cys | = | L-Cysteine |
| Arg | = | L-Arginine |
| Gln | = | L-Glutamine |
| Pro | = | L-Proline |
| Leu | = | L-Leucine |
| Met | = | L-Methionine |
| Ser | = | L-Serine |
| Asn | = | L-Asparagine |
| His | = | L-Histidine |
| Trp | = | L-Tryptophan |
| Val | = | L-Valine |
| iLys | = | $N^{\epsilon}$-Isopropyl-L-lysine |
| 4-Abu | = | 4-Aminobutyric acid |
| Nle | = | Norleucine |
| Sar | = | Sarcosine |
| Sar-ol | = | Sarcosine Alcohol |
| Gly-ol | = | 2-Aminoethanol |
| Met(O) | = | Methionine Sulfoxide |
| Orn | = | L-Ornithine |
| $D^{\alpha}$Nal | = | $\alpha$-naphthyl-D-alanine |
| $D^{\beta}$Nal | = | $\beta$-naphthyl-D-alanine |

All three letter amino acid abbreviations preceded by a "D" indicate the D-configuration of the amino acid residue;
Group 3 polypeptides are selected from any of the polypetides having the structure:

Tyr-DArg-Phe-$NH_2$;
Tyr-DAla-Phe-$NH_2$;
Tyr-DArg($NO_2$)-Phe-$NH_2$;
Tyr-DMet(O)-Phe-$NH_2$;
Tyr-DAla-Phe-Gly-$NH_2$;
Tyr-DArg-Phe-Gly-$NH_2$;
Tyr-DThr-Phe-Gly-$NH_2$;
Phe-DArg-Phe-Gly-$NH_2$;
Tyr-DArg-Phe-Sar;
Tyr-DAla-Gly-Phe-$NH_2$;
Tyr-DArg-Gly-Trp-$NH_2$;
Tyr-DArg($NO_2$)-Phe-Gly-$NH_2$;
Tyr-DMet(O)-Phe-Gly-$NH_2$;
(NMe)Tyr-DArg-Phe-Sar-$NH_2$;
Tyr-DArg-Phe-Gly-ol;
Tyr-DArg-Gly-(NMe)Phe-$NH_2$;
Tyr-DArg-Phe-Sar-ol;
Tyr-DAla-Phe-Sar-ol;
Tyr-DAla-Phe-Gly-Tyr-$NH_2$;
Gly-Tyr-DArg-Phe-Gly-$NH_2$;

Tyr-DThr-Gly-Phe-Thz-$NH_2$;
Gly-Tyr-DAla-Phe-Gly-$NH_2$;
Tyr-DAla-Phe-Gly-ol;
Tyr-DAla-Gly-(NMe)Phe-Gly-ol;
Tyr-DArg-Phe-Sar-$NH_2$;
Tyr-DAla-Phe-Sar-$NH_2$;
Tyr-DAla-Phe-Sar;
Tyr-DAla-Gly-(NMe)Phe-$NH_2$;
Sar-Tyr-DArg-Phe-Sar-$NH_2$;
Tyr-DCys-Phe-Gly-DCys-$NH_2$ (cyclic disulfide);
Tyr-DCys-Phe-Gly-DCys-$NH_2$ (free dithiol);
Tyr-DCys-Gly-Phe-DCys-$NH_2$ (cyclic disulfide);
Tyr-DCys-Gly-Phe-DCys-$NH_2$ (free dithiol);
Tyr-DAla-Phe-Gly-Tyr-Pro-Ser-$NH_2$;
Tyr-DAla-Phe-Sar-Tyr-Pro-Ser-$NH_2$;
Tyr-DAla-Phe-Sar-Phe-Pro-Ser-$NH_2$;
Tyr-DAla-Phe-Gly-Tyr-Hyp-Ser-$NH_2$;
Tyr-DAla-Phe-Sar-Tyr-Hyp-Ser-$NH_2$;
Tyr-DAla-Phe-Sar-Phe-Hyp-Ser-$NH_2$;
Tyr-DArg-Phe-Gly-Tyr-Hyp-Ser-$NH_2$;
Tyr-DArg-Phe-Sar-Tyr-Pro-Ser-$NH_2$;
Tyr-DArg-Phe-Sar-Tyr-Hyp-Ser-$NH_2$;
Tyr-DArg-Phe-Gly-Tyr-Pro-Ser-$NH_2$;
and
organic or inorganic addition salts of any of said polypeptides of Group 3.

**15.** Method of Claim 14 wherein said Group 1 polypeptides are selected from any of the polypeptides:
(a) having the following amino acid sequences in Positions 1-44 (numbered from N terminus to C terminus):
(#144) YADAIFTNSYRKVLGQLSARKLLQDIMSRQQGESNQERGARARL-X,
(#145) YADAIFTNSYRKVLGQLSARKLLQDIMSRQQGERNQEQGARVRL-X,
(#146) YADAIFTNSYRKVLGQLSARKLLQDIMNRQQGERNQEQGAKVRL-X,
(#148) YADAIFTNSYRKILGQLSARKLLQDIMNRQQGERNQEQGAKVRL-X,
(#149) HADAIFTSSYRRILGQLYARKLLHEIMNRQQGERNQEQRSRFN-X; and functional equivalents thereof;
wherein the C-terminal amino acid has the following truncated general formula:

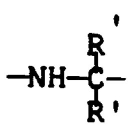

wherein each R' independently represents the substituents of the particular amino acid residue, e.g.; hydrogen, alkyl, aryl, amino or acid substituents; X denotes the C terminal end group and is selected from -$CONH_2$, -COOH, -COOR, -CONRR, -$CH_2OH$, and -$CH_2OR$, where R is an alkyl group having 1-6 carbon atoms or an aromatic ring having up to 12 carbon atoms or an aromatic ring having up to 12 carbon atoms; and wherein the amino acid residue abbreviations used are in accordance with the standard peptide nomenclature:

G = Gly (Glycine),
Y = Tyr (L-Tyrosine),
I = Ile (L-Isoleucine),
E = Glu (L-Glutamic Acid),
T = Thr (L-Threonine),
F = Phe (L-Phenylalanine),
A = Ala (L-Alanine),
K = Lys (L-Lysine),
D = Asp (L-Aspartic Acid),

| | | |
|---|---|---|
| C | = | Cys (L-Cysteine), |
| R | = | Arg (L-Arginine), |
| Q | = | Gln (L-Glutamine), |
| P | = | Pro (L-Proline), |
| L | = | Leu (L-Leucine), |
| M | = | Met (L-Methionine), |
| S | = | Ser (L-Serine), |
| N | = | Asn (L-Asparagine), |
| H | = | His (L-Histidine), |
| W | = | Trp (L-Tryptophan), and |
| V | = | Val (L-Valine); |
| Aib | = | $\alpha$-Aminoisobutyric Acid |
| Nle | = | Norleucine |
| (NMe)DAla | = | N-Methyl-D-Alanine |

(b) any one of said (a) polypeptides having the following amino acid substitutions:
Position 1 of (#144-#148) is DTyr or His;
Position 1 of (#149) is Tyr or DHis;
Position 2 of (#144-#149) is (NMe)DAla or Aib or DAla;
Position 3 of (#144-#149) is DAsp;
Position 4 of (#144-#149) is DAla; and
Position 1 + 2 of (#144-#149) is:
$DTyr^1 + DAla^2$, $DTyr^1 + (NMe)DAla^2$, or $DTyr^1 + Aib^2$;
(c) any one of said (a) or (b) polypeptides having a substitution of Nle for Met at Position 27;
(d) any one of said (a), (b) or (c) polypeptides in which the N-terminus $-NH_2$ is replaced by -NHCOR and wherein R is an alkyl group having 1 to 6 carbon atoms, or an aromatic ring having up to 12 carbon atoms;
(e) fragments of any one of said (a), (b), (c) or (d) polypeptides which contain at least the amino acid residues of Positions 1-29;
(f) having the following specific amino acid sequences in Positions 1-29 (numbered from N terminus to C terminus):
YADAIFTNSYRKVLQQLAARKLLQDIMSR-X,
YADAIFTNSYRKVLQQLLARKLLQDIMSR-X,
YSDAIFSNAYRKILQQLLARKLLQDIMOR-X,
YADAIFSNAYRKILQQLLARKLLQDIMQR-X,
YADAIFSSAYRRLLAQLASRRLLQELLAR-X,
YADAIFTNCYRKVLCQLSARKLLQDIMSR-X (linear dithiol), and
YADAIFTNCYRKVLCQLSARKLLQDIMSR-X (cyclic disulfide);
wherein the C-terminal amino acid and X are as defined above; and modification of any one of these group (f) compounds in accordance with the modifications set forth in (b), (c) and (d) above; and
(g) organic or inorganic addition salts of any of said (a), (b), (c), (d), (e) or (f) polypeptides of Group 1.

**16.** Method of Claim 14 wherein said combination comprises a compound from each of Group 1 polypeptides and Group 2 polypeptides.

**17.** Method of Claim 14 wherein said combination comprises a compound from each of Group 2 polypeptides and Group 3 polypeptides.

**18.** Method of Claim 14 wherein said combination comprises a compound from each of Group 1 polypeptides, Group 2 polypeptides and Group 3 polypeptides.

**19.** A pharmaceutical preparation which comprises an effective amout of the compound of any of claims 1 to 3 and an inert carrier.

**20.** A pharmaceutical preparation which comprises an effective amount of the combination of any of claims 9 to 13 and an inert carrier.

## Patentansprüche

1. Polypeptid, das in der Lage ist, die Freisetzung und Erhöhung von Wachstumshormonspiegeln in dem Blut eines Empfängertieres zu fördern, wobei das Polypeptid ausgewählt ist aus der Gruppe, die aus Polypeptiden besteht, die definiert sind durch die allgemeine Struktur:

   AA1-AA2-AA3-Trp-AA5-AA6-AA7-Z,

   wobei AA1 ausgewählt ist aus der Gruppe bestehend aus His und 3(NMe)His (d.h., wobei der Imidazolring an der 3-Position methyliert ist); AAO-His und AAO-3(NMe)His; wobei AAO ausgewählt ist aus der Gruppe bestehend aus jeder natürlich vorkommenden L-Aminosäure, Met(O), DOPA und Abu;
   AA2 ausgewählt ist aus der Gruppe bestehend aus DPhe, DTrp, 5-Fluoro-D oder D/LTrp; 6-Fluoro-D oder D/LTrp (d.h., wobei der Indolring an der 5- oder 6-Position fluoriert ist), (Formyl)DTrp (d.h. DTrp, das am Indolstickstoff formyliert ist), *XTrp, wobei *XTrp ausgewählt ist aus der Gruppe bestehend aus den N-monomethylierten DTrp-Isomeren (d.h., ($N^{\alpha}$Me)DTrp und (Indol NMe)DTrp), $D^{\alpha}$Nal und $D^{\beta}$Nal;
   AA3 ausgewählt ist aus der Gruppe bestehend aus Ala, Gly und Ser;
   AA5 ausgewählt ist aus der Gruppe bestehend aus DPhe und (NMe)DPhe;
   AA6 ausgewählt ist aus der Gruppe bestehend aus allen natürlich vorkommenden L-Aminosäuren, Dipeptiden der natürlich vorkommenden Aminosäuren, z.B. Ala-Ala, und Verbindungen der Formel:

   $H_2N-(CH_2)_n-CO_2H$,

   wobei n = 2-12;
   AA7 ausgewählt ist aus der Gruppe bestehend aus Arg, iLys, Lys und Orn;
   Z die C-terminale Endgruppe des Polypeptides oder die C-terminale(n) Aminosäure(n) plus Endgruppe darstellt, wobei Z ausgewählt ist aus der Gruppe bestehend aus $-CONH_2$, -COOH, -COOR, -CONHR, $-CONR_2$, $-CH_2OH$ und $-CH_2OR$, wobei R eine Alkylgruppe mit 1-6 Kohlenstoffatomen oder ein aromatischer Ring mit bis zu 12 Kohlenstoffatomen ist; und wobei Z alternativ ausgewählt ist aus der Gruppe bestehend aus -Gly-Z', -Met-Z', -Lys-Z', -Cys-Z', -Gly-Tyr-Z' und Ala-Tyr-Z', wobei Z' ausgewählt ist aus der Gruppe bestehend aus $-CONH_2$, -COOH, -CONHR, -COOR, $-CONR_2$, $-CH_2OH$ und $-CH_2OR$, wobei R wie vorstehend definiert ist;
   und organischen oder anorganischen Additionssalzen jedes der Polypeptide;
   wobei die verwendeten Aminosäurerestabkürzungen mit der Standardpeptidnomenklatur übereinstimmen:

| | | |
|---|---|---|
| Gly | = | Glycin |
| Tyr | = | L-Tyrosin |
| Ile | = | L-Isoleucin |
| Glu | = | L-Glutaminsäure |
| Thr | = | L-Threonin |
| Phe | = | L-Phenylalanin |
| Ala | = | L-Alanin |
| Lys | = | L-Lysin |
| Asp | = | L-Asparaginsäure |
| Cys | = | L-Cystein |
| Arg | = | L-Arginin |
| Gln | = | L-Glutamin |
| Pro | = | L-Prolin |
| Leu | = | L-Leucin |
| Met | = | L-Methionin |
| Ser | = | L-Serin |
| Asn | = | L-Asparagin |
| His | = | L-Histidin |
| Trp | = | L-Tryptophan |
| Val | = | L-Valin |
| DOPA | = | 3,4-Dihydroxyphenylalanin |
| Met(O) | = | Methioninsulfoxid |
| Abu | = | $\alpha$-Aminobuttersäure |
| iLys | = | $N^{\epsilon}$-Isopropyl-L-Lysin |
| 4-Abu | = | 4-Aminobuttersäure |
| Orn | = | L-Ornithin |

$D^{\alpha}Nal$ = $\alpha$-Naphthyl-D-Alanin
$D^{\beta}Nal$ = $\beta$-Naphthyl-D-Alanin
Alle Drei-Buchstaben-Aminosäureabkürzungen, denen ein "D" vorangestellt ist, bezeichnen die D-Konfiguration des Aminosäurerestes; Abkürzungen, denen ein "D/L" vorangestellt ist, bezeichnen eine Mischung der D- und L-Konfigurationen der bezeichneten Aminosäuren; und Glycin ist im Umfang des Begriffes "natürlich vorkommende L-Aminosäuren" eingeschlossen.

**2.** Polypeptid gemäß Anspruch 1, wobei das Polypeptid ausgewählt ist aus der Gruppe bestehend aus:
AA1-AA2-AA3-TRP-AA5-Ala-AA7-$NH_2$;
AA1-AA2-AA3-Trp-AA5-Ala-Ala-AA7-$NH_2$;
und
organischen oder anorganischen Additionssalzen jedes der Polypeptide.

**3.** Polypeptid gemäß Anspruch 1, wobei das Polypeptid ausgewählt ist aus der Gruppe bestehend aus:
His-DTrp-Ala-Trp-DPhe-Ala-Lys-$NH_2$;
His-DTrp-Ala-Trp-DPhe-Ala-Ala-Lys-$NH_2$; und
organischen oder anorganischen Additionssalzen jedes der beiden Polypeptide.

**4.** Verfahren zur Förderung der Freisetzung und Erhöhung der Blutwachstumshormonspiegel bei Tieren durch Verabreichung einer wirksamen Menge wenigstens eines der in einem der Ansprüche 1, 2 oder 3 dargelegten Polypeptide an diese; ausschließlich jedes Verfahrens zur therapeutischen Behandlung des menschlichen oder tierischen Körpers.

**5.** Verbindung der Formel:

AA1-AA2-AA3-Trp-AA5-AA6-AA7-Ⓡ

wobei AA1 ausgewählt ist aus der Gruppe bestehend aus His und 3(NMe)His, (d.h. wobei der Imidazolring an der 3-Position methyliert ist); AAO-His, AAO-3(NMe)His; wobei AAO jede natürlich vorkommende L-Aminosäure ist;
AA2 ausgewählt ist aus der Gruppe bestehend aus DPhe, DTrp, 5-Fluoro-D oder D/LTrp; 6-Fluoro-D oder D/LTrp (d.h., wobei der Indolring an der 5- oder 6-Position fluoriert ist), (Formyl)DTrp (d.h. DTrp, das an dem Indolstickstoff formyliert ist) *XTrp, wobei *XTrp ausgewählt ist aus der Gruppe bestehend aus den N-monomethylierten DTrp-Isomeren, (d.h. $N^{\alpha}Me$)DTrp und (Indol NMe)DTrp), $D^{\alpha}Nal$ und $D^{\beta}Nal$;
AA3 ausgewählt ist aus der Gruppe bestehend aus Ala, Gly und Ser;
AA5 ausgewählt ist aus der Gruppe bestehend aus DPhe und (NMe)DPhe;
AA6 ausgewählt ist aus der Gruppe bestehend aus allen natürlich vorkommenden L-Aminosäuren, Dipeptiden der natürlich vorkommenden Aminosäuren, z.B. Ala-Ala, und Verbindungen der Formel:

$H_2N(CH_2)_nCO_2H$

wobei n = 2-12;
AA7 ausgewählt ist aus der Gruppe bestehend aus Arg, iLys Lys und Orn;
und wobei Ⓡ ein Polymerharz ist und funktionelle Gruppen der die Bestandteile bildenden Aminosäuren mit geeigneten Schutzgruppen nach Bedarf geschützt sind.

**6.** Verbindung der Formel:

His-DTrp-Ala-Trp-DPhe-Ala-Lys-Ⓡ

wobei Ⓡ ein Polymerharz ist und funktionelle Gruppen der die Bestandteile bildenden Aminosäuren mit geeigneten Schutzgruppen nach Bedarf geschützt sind.

**7.** Verbindung der Formel:

His-DTrp-Ala-Trp-DPhe-Ala-Ala-Lys-Ⓡ

wobei Ⓡ ein Polymerharz ist und funktionelle Gruppen der die Bestandteile bildenden Aminosäuren

mit geeigneten Schutzgruppen nach Bedarf geschützt sind.

**8.** Harzgebundenes Polypeptid, ausgewählt aus der Gruppe bestehend aus:
DTrp-Ala-Trp-DPhe-Ala-Ala-Lys-Ⓡ,
DTrp-Ala-Trp-DPhe-Ala-Lys-Ⓡ,
Ala-Trp-DPhe-Ala-Ala-Lys-Ⓡ,
Ala-Trp-DPhe-Ala-Lys-Ⓡ,
Trp-DPhe-Ala-Ala-Lys-Ⓡ,
Trp-DPhe-Ala-Lys-Ⓡ,
DPhe-Ala-Ala-Lys-Ⓡ, und
DPhe-Ala-Lys-Ⓡ,
wobei Ⓡ ein Polymerharz ist und funktionelle Gruppen der die Bestandteile bildenden Aminosäuren mit geeigneten Schutzgruppen nach Bedarf geschützt sind.

**9.** Zusammensetzung, die zur Verursachung der Freisetzung und Erhöhung des Spiegels von Wachstumshormon im Blut eines Tieres wirksam ist, wobei die Zusammensetzung eine wirksame Menge von Polypeptiden umfaßt, die ausgewählt sind aus wenigstens zwei verschiedenen Gruppen der Polypeptide der Gruppe 1, Polypeptide der Gruppe 2 oder Polypeptide der Gruppe 3;
wobei die Polypeptide der Gruppe 1 ausgewählt sind aus jedem der natürlich vorkommenden wachstumshormon-Releasinghormone und funktionellen Äquivalenten derselben, wobei die Polypeptide an dem wachstumshormon-Releasinghormonrezeptor von Säugetieren und anderen Wirbeltieren sowie Krustentieren wirken;
Polypeptide der Gruppe 2 ausgewählt sind aus jedem der Polypeptide mit der Struktur:
AA1-AA2-AA3-Trp-AA5-AA6-AA7-Z,
wobei AA1 ausgewählt ist aus der Gruppe bestehend aus His und 3(NMe)His (d.h., wobei der Imidazolring an der 3-Position methyliert ist); AAO-His und AAO-3(NMe)His; wobei AAO jede natürlich vorkommende L-Aminosäure ist;
AA2 ausgewählt ist aus der Gruppe bestehend aus DPhe, DTrp, 5-Fluoro-D oder D/LTrp; 6-Fluoro-D oder D/LTrp (d.h., wobei der Indolring an der 5- oder 6-Position fluoriert ist), (Formyl)DTrp (d.h. DTrp, das am Indolstickstoff formyliert ist), *XTrp, wobei *XTrp ausgewählt ist aus der Gruppe bestehend aus den N-monomethylierten DTrp-Isomeren (d.h. ($N^{\alpha}$Me)DTrp und (Indol NMe)DTrp), $D^{\alpha}$Nal und $D^{\beta}$Nal;
AA3 ausgewählt ist aus der Gruppe bestehend aus Ala, Gly und Ser;
AA5 ausgewählt ist aus der Gruppe bestehend aus DPhe und (NMe)DPhe;
AA6 ausgewählt ist aus der Gruppe bestehend aus allen natürlich vorkommenden L-Aminosäuren, Dipeptiden der natürlich vorkommenden L-Aminosäuren, z.B. Ala-Ala, und Verbindungen der Formel:

$H_2N-(CH_2)_n-CO_2H$,

wobei n = 2-12;
AA7 ausgewählt ist aus der Gruppe bstehend aus Arg, iLys, Lys und Orn;
Z die C-terminale Endgruppe des Polypeptids oder die C-terminale(n) Aminosäure(n) plus Endgruppe darstellt, wobei Z ausgewählt ist aus der Gruppe bestehend aus $-CONH_2$, -COOH, -COOR, -CONHR, $-CONR_2$, $-CH_2OH$ und $-CH_2OR$, wobei R eine Alkylgruppe mit 1-6 Kohlenstoffatomen oder ein aromatischer Ring mit bis zu 12 Kohlenstoffatomen ist; und wobei Z alternativ ausgewählt ist aus der Gruppe bestehend aus -Gly-Z', -Met-Z', -Lys-Z', -Cys-Z', -Gly-Tyr-Z' und Ala-Tyr-Z', wobei Z' ausgewählt ist aus der Gruppe bestehend aus $-CONH_2$, -COOH, -CONHR, -COOR, $-CONR_2$, $-CH_2OH$ und $-CH_2OR$, wobei R wie vorstehend definiert ist;
und organischen oder anorganischen Additionssalzen jedes der Polypeptide;
wobei die verwendeten Aminosäurerestabkürzungen mit der Standardpeptidnomenklatur übereinstimmen:

| | | |
|---|---|---|
| Gly | = | Glycin |
| Tyr | = | L-Tyrosin |
| Ile | = | L-Isoleucin |
| Glu | = | L-Glutaminsäure |
| Thr | = | L-Threonin |
| Phe | = | L-Phenylalanin |
| Ala | = | L-Alanin |
| Lys | = | L-Lysin |

Asp = L-Asparaginsäure
Cys = L-Cystein
Arg = L-Arginin
Gln = L-Glutamin
Pro = L-Prolin
Leu = L-Leucin
Met = L-Methionin
Ser = L-Serin
Asn = L-Asparagin
His = L-Histidin
Trp = L-Tryptophan
Val = L-Valin
DOPA = 3,4-Dihydroxyphenylalanin
Gly-ol = 2-Aminoethanol
Hyp = trans-4-Hydroxy-L-Prolin
Met(O) = Methioninsulfoxid
Met(O)-ol = Methioninsulfoxidalkohol
Sar = Sarkosin
Sar-ol = Sarkosinalkohol
Thz = L-Thiazolidin-4-Karbonsäure
iLys = $N^{\epsilon}$-Isopropyl-L-Lysin
4-Abu = 4-Aminobuttersäure
Orn = L-Ornithin
$D^{\alpha}$Nal = $\alpha$-Naphthyl-D-Alanin
$D^{\beta}$Nal = $\beta$-Naphthyl-D-Alanin

Alle Drei-Buchstaben-Aminosäureabkürzungen, denen ein "D" vorangestellt ist, bezeichnen die D-Konfiguration des Aminosäurerestes; Abkürzungen, denen ein "D/L" vorangestellt ist, bezeichnen eine Mischung der D- und L-Konfigurationen der bezeichneten Aminosäuren; und Glycin ist im Umfang des Begriffes "natürlich vorkommende L-Aminosäuren" eingeschlossen; und

Polypeptide der Gruppe 3 ausgewählt sind aus einem der Polypeptide, das die Struktur hat:

Tyr-DArg-Phe-$NH_2$;
Tyr-DAla-Phe-$NH_2$;
Tyr-DArg($NO_2$)-Phe-$NH_2$;
Tyr-DMet(O)-Phe-$NH_2$;
Tyr-DAla-Phe-Gly-$NH_2$;
Tyr-DArg-Phe-Gly-$NH_2$;
Tyr-DThr-Phe-Gly-$NH_2$;
Phe-DArg-Phe-Gly-$NH_2$;
Tyr-DArg-Phe-Sar;
Tyr-DAla-Gly-Phe-$NH_2$;
Tyr-DArg-Gly-Trp-$NH_2$;
Tyr-DArg-($NO_2$)-Phe-Gly-$NH_2$;
Tyr-DMet(O)-Phe-Gly-$NH_2$;
(NMe)Tyr-DArg-Phe-Sar-$NH_2$;
Tyr-DArg-Phe-Gly-ol;
Tyr-DArg-Gly-(NMe)Phe-$NH_2$;
Tyr-DArg-Phe-Sar-ol;
Tyr-DAla-Phe-Sar-ol;
Tyr-DAla-Phe-Gly-Tyr-$NH_2$;
Gly-Tyr-DArg-Phe-Gly-$NH_2$;
Tyr-DThr-Gly-Phe-Thz-$NH_2$;
Gly-Tyr-DAla-Phe-Gly-$NH_2$;
Tyr-DAla-Phe-Gly-ol;
Tyr-DAla-Gly-(NMe)Phe-Gly-ol;
Tyr-DArg-Phe-Sar-$NH_2$;
Tyr-DAla-Phe-Sar-$NH_2$;
Tyr-DAla-Phe-Sar;
Tyr-DAla-Gly-(NMe)Phe-$NH_2$;

Sar-Tyr-DArg-Phe-Sar-$NH_2$;
Tyr-DCys-Phe-Gly-DCys-$NH_2$ (cyclisches Disulfid);
Tyr-DCys-Phe-Gly-DCys-$NH_2$ (freies Dithiol);
Tyr-DCys-Gly-Phe-DCys-$NH_2$ (cyclisches Disulfid);
Tyr-DCys-Gly-Phe-DCys-$NH_2$ (freies Dithiol);
Tyr-DAla-Phe-Gly-Tyr-Pro-Ser-$NH_2$;
Tyr-DAla-Phe-Sar-Tyr-Pro-Ser-$NH_2$;
Tyr-DAla-Phe-Sar-Phe-Pro-Ser-$NH_2$;
Tyr-DAla-Phe-Gly-Tyr-Hyp-Ser-$NH_2$;
Tyr-DAla-Phe-Sar-Tyr-Hyp-Ser-$NH_2$;
Tyr-DAla-Phe-Sar-Phe-Hyp-Ser-$NH_2$;
Tyr-DArg-Phe-Gly-Tyr-Hyp-Ser-$NH_2$;
Tyr-DArg-Phe-Sar-Tyr-Pro-Ser-$NH_2$;
Tyr-DArg-Phe-Sar-Tyr-Hyp-Ser-$NH_2$;
Tyr-DArg-Phe-Gly-Tyr-Pro-Ser-$NH_2$;
und organischen oder anorganischen Additionssalzen jedes der Polypeptide der Gruppe 3;

wobei die Zusammensetzung in einem solchen Verhältnis verabreicht wird, daß die Zusammensetzung zur Verursachung der synergistischen Freisetzung und Erhöhung von Wachstumshormon im Blut des entsprechenden Tieres wirksam ist.

**10.** Zusammensetzung nach Anspruch 9, wobei die Polypeptide der Gruppe 1 ausgewählt sind aus den Polypeptiden:

(a) mit den folgenden Aminosäuresequenzen an den Positionen 1-44 (numeriert vom terminalen N bis zum terminalen C):

(#144) YADAIFTNSYRKVLGQLSARKLLQDIMSRQQGESNQERGARARL-X,
(#145) YADAIFTNSYRKVLGQLSARKLLQDIMSRQQGERNQEQGARVRL-X,
(#146) YADAIFTNSYRKVLGQLSARKLLQDIMNRQQGERNQEQGAKVRL-X,
(#148) YADAIFTNSYRKILGQLSARKLLQDIMNRQQGERNQEQGAKVRL-X,
(#149) HADAIFTSSYRRILGQLYARKLLHEIMNRQQGERNQEQRSRFN-X; und funktionelle Äquivalente davon;

wobei die C-terminale Aminosäure die folgende allgemeine Rumpfformel hat:

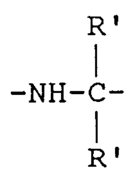

wobei jedes R' unabhängig die Substituenten des speziellen Aminosäurerestes, z.B. Wasserstoff-, Alkyl-, Aryl-, Amino- oder Säuresubstituenten darstellt; X die C-terminale Endgruppe bezeichnet und ausgewählt ist aus $-CONH_2$, -COOH, -COOR, -CONRR, $-CH_2OH$ und $-CH_2OR$, wobei R eine Alkylgruppe mit 1-6 Kohlenstoffatomen oder ein aromatischer Ring mit bis zu 12 Kohlenstoffatomen ist; und wobei die verwendeten Aminosäurerestabkürzungen mit der Standardpeptidnomenklatur übereinstimmen:

| | |
|---|---|
| G | = Gly (Glycin) |
| Y | = Tyr (L-Tyrosin) |
| I | = Ile (L-Isoleucin) |
| E | = Glu (L-Glutaminsäure) |
| T | = Thr (L-Threonin) |
| F | = Phe (L-Phenylalanin) |
| A | = Ala (L-Alanin) |
| K | = Lys (L-Lysin) |
| D | = Asp (L-Asparaginsäure) |
| C | = Cys (L-Cystein) |
| R | = Arg (L-Arginin) |
| Q | = Gln (L-Glutamin) |

P = Pro (L-Prolin)
L = Leu (L-Leucin)
M = Met (L-Methionin)
S = Ser (L-Serin)
N = Asn (L-Asparagin)
H = His (L-Histidin)
W = Trp (L-Trytophan)
V = Val (L-Valin)
Nle = Norleucin
Sar = Sarkosin
Sar-ol = Sarkosinalkohol
Gly-ol = 2-Aminoethanol
Met(O) = Methioninsulfoxid

(b) jedem einzelnen der Polypeptide aus (a), das die folgenden Aminosäuresubstitutionen hat:
Position 1 von (#144-#148) ist DTyr oder His;
Position 1 von (#149) ist Tyr oder DHis;
Position 2 von (#144-#149) ist (NMe)Dala oder Aib oder DAla;
Position 3 von (#144-#149) ist DAsp;
Position 4 von (#144-#149) ist DAla; und
Position 1 + 2 von (#144-#149) ist:
$DTyr^1$ + $DAla^2$, $DTyr^1$ + $(NMe)DAla^2$, oder $DTyr^1$ + $Aib^2$;
(c) jedem einzelnen der Polypeptide nach (a) oder (b), das eine Substitution von Nle für Met an der Position 27 hat;
(d) jedem einzelnen der Polypeptide nach (a), (b) oder (c), bei dem die N-terminale $-NH_2$-Gruppe durch -NHCOR ersetzt ist und wobei R eine Alkylgruppe mit 1-6 Kohlenstoffatomen oder ein aromatischer Ring mit bis zu 12 Kohlenstoffatomen ist;
(e) Fragmenten jedes einzelnen der Polypeptide nach (a), (b), (c) oder (d), die wenigsten die Aminosäurereste der Positionen 1 bis 29 enthalten;
(f) mit den folgenden bestimmten Aminosäuresequenzen an Positionen 1 bis 29 (numeriert vom terminalen N zum terminalen C):
YADAIFTNSYRKVLQQLAARKLLQDIMSR-X,
YADAIFTNSYRKVLQQLLARKLLQDIMSR-X,
YSDAIFSNAYRKILQQLLARKLLQDIMOR-X,
YADAIFSNAYRKILQQLLARKLLQDIMQR-X,
YADAIFSSAYRRLLAQLASRRLLQELLAR-X,
YADAIFTNCYRKVLCQLSARKLLQDIMSR-X (lineares Dithiol), und
YADAIFTNCYRKVLCQLSARKLLQDIMSR-X (cyclisches Disulfid);
wobei die C-terminale Aminosäure und X wie vorstehend definiert sind; und Modifikationen jeder einzelnen dieser Verbindungen nach Gruppe (f) in Übereinstimmung mit den vorstehend unter (b), (c) und (d) dargelegten Modifikationen; und
(g) organischen oder anorganischen Additionssalzen jedes der Polypeptide der Gruppe 1 nach (a), (b), (c), (d), (e) oder (f).

**11.** Zusammensetzung nach Anspruch 9, umfassend eine Verbindung aus jeweils den Polypeptiden der Gruppe 1 und den Polypeptiden der Gruppe 2.

**12.** Zusammensetzung nach Anspruch 9, umfassend eine Verbindung aus jeweils Polypeptiden der Gruppe 2 und Polypeptiden der Gruppe 3.

**13.** Zusammensetzung nach Anspruch 9, umfassend eine Verbindung aus jeweils Polypeptiden der Gruppe 1, Polypeptiden der Gruppe 2 und Polypeptiden der Gruppe 3.

**14.** Verfahren zur Verursachung der Freisetzung und Erhöhung des Spiegels von Wachstumshormon im Blut eines Tieres; ausschließlich jedes Verfahrens zur therapeutischen Behandlung des menschlichen oder tierischen Körpers, umfassend die Verabreichung einer wirksamen Dosis einer Zusammensetzung, die Polypeptide umfaßt, die ausgewählt sind aus wenigstens zwei verschiedenen Gruppen aus Polypeptiden der Gruppe 1, Polypeptiden der Gruppe 2 oder Polypeptiden der Gruppe 3;
wobei Polypeptide der Gruppe 1 ausgewählt sind aus jedem der natürlich vorkommenden Wachstumshormon Releasinghormone und funktionellen Äquivalenten derselben, wobei die Polypeptide an dem Wachstumshormon-Releasinghormonrezeptor von Säugetieren und anderen Wirbeltieren sowie Krustentieren wirken;

Polypeptide der Gruppe 2 ausgewählt sind aus jedem beliebigen der Polypeptide mit der Struktur:

AA1-AA2-AA3-Trp-AA5-AA6-AA7-Z,

wobei AA1 ausgewählt ist aus der Gruppe bestehend aus His und 3(NMe)His (d.h., wobei der Imidazolring an der 3-Position methyliert ist); AAO-His und AAO-3(NMe)His; wobei AAO jede natürlich vorkommende L-Aminosäure ist;

AA2 ausgewählt ist aus der Gruppe bestehend aus DPHe, DTrp, 5-Fluoro-D oder D/LTrp; 6-Fluoro-D oder D/LTrp (d.h., wobei der Indolring an der 5- oder 6-Position fluoriert ist), (Formyl)DTrp (d.h. DTrp, das am Indolstickstoff formyliert ist), *XTrp, wobei *XTrp ausgewählt ist aus der Gruppe bestehend aus den N-monomethylierten DTrp-Isomeren (d.h. ($N^{\alpha}$Me)DTrp und (Indol NMe)DTrp),$D^{\alpha}$Nal und $D^{\beta}$Nal;

AA3 ausgewählt ist aus der Gruppe bestehend aus Ala, Gly und Ser;

AA5 ausgewählt ist aus der Gruppe bestehend aus DPhe und (NMe)DPhe;

AA6 ausgewählt ist aus der Gruppe bestehend aus allen natürlich vorkommenden L-Aminosäuren, Dipeptiden der natürlich vorkommenden L-Aminosäuren, z.B. Ala-Ala, und Verbindungen der Formel:

$H_2N-(CH_2)_n-CO_2H$,

wobei n = 2-12;

AA7 ausgewählt ist aus der Gruppe bstehend aus Arg, iLys, Lys und Orn;

Z die C-terminale Endgruppe des Polypeptids oder die C-terminale(n) Aminosäure(n) plus Endgruppe darstellt, wobei Z ausgewählt ist aus der Gruppe bestehend aus $-CONH_2$, -COOH, -COOR, -CONHR, $-CONR_2$, $-CH_2OH$ und $-CH_2OR$, wobei R eine Alkylgruppe mit 1-6 Kohlenstoffatomen oder ein aromatischer Ring mit bis zu 12 Kohlenstoffatomen ist; und wobei Z alternativ ausgewählt ist aus der Gruppe bestehend aus -Gly-Z', -Met-Z', -Lys-Z', -Cys-Z', -Gly-Tyr-Z' und Ala-Tyr-Z', wobei Z' ausgewählt ist aus der Gruppe bestehend aus $-CONH_2$, -COOH, -CONHR, -COOR, $-CONR_2$, $-CH_2OH$ und $-CH_2OR$, wobei R wie vorstehend definiert ist;

und organischen oder anorganischen Additionssalzen jedes der Polypeptide;

wobei die verwendeten Aminosäurerestabkürzungen mit der Standardpeptidnomenklatur übereinstimmen:

| | | |
|---|---|---|
| Gly | = | Glycin |
| Tyr | = | L-Tyrosin |
| Ile | = | L-Isoleucin |
| Glu | = | L-Glutaminsäure |
| Thr | = | L-Threonin |
| Phe | = | L-Phenylalanin |
| Ala | = | L-Alanin |
| Lys | = | L-Lysin |
| Asp | = | L-Asparaginsäure |
| Cys | = | L-Cystein |
| Arg | = | L-Arginin |
| Gln | = | L-Glutamin |
| Pro | = | L-Prolin |
| Leu | = | L-Leucin |
| Met | = | L-Methionin |
| Ser | = | L-Serin |
| Asn | = | L-Asparagin |
| His | = | L-Histidin |
| Trp | = | L-Tryptophan |
| Val | = | L-Valin |
| iLys | = | $N^{\epsilon}$-Isopropyl-L-Lysin |
| 4-Abu | = | 4-Aminobuttersäure |
| Nle | = | Norleucin |
| Sar | = | Sarkosin |
| Sar-ol | = | Sarkosinalkohol |
| Gly-ol | = | 2-Aminoethanol |
| Met(O) | = | Methioninsulfoxid |

Orn = L-Ornithin
$D^{\alpha}$Nal = $\alpha$-Naphthyl-D-Alanin
$D^{\beta}$Nal = $\beta$-Naphthyl-D-Alanin

Alle Drei-Buchstaben-Aminosäureabkürzungen, denen ein "D" vorangestellt ist, bezeichnen die D-Konfiguration des Aminosäurerestes;

Polypeptide der Gruppe 3 ausgewählt sind aus jedem der Polypeptide, das die Struktur hat:

Tyr-DArg-Phe-$NH_2$;
Tyr-DAla-Phe-$NH_2$;
Tyr-DArg($NO_2$)-Phe-$NH_2$;
Tyr-DMet(O)-Phe-$NH_2$;
Tyr-DAla-Phe-Gly-$NH_2$;
Tyr-DArg-Phe-Gly-$NH_2$;
Tyr-DThr-Phe-Gly-$NH_2$;
Phe-DArg-Phe-Gly-$NH_2$;
Tyr-DArg-Phe-Sar;
Tyr-DAla-Gly-Phe-$NH_2$;
Tyr-DArg-Gly-Trp-$NH_2$;
Tyr-DArg($NO_2$)-Phe-Gly-$NH_2$;
Tyr-DMet(O)-Phe-Gly-$NH_2$;
(NMe)Tyr-DArg-Phe-Sar-$NH_2$;
Tyr-DArg-Phe-Gly-ol;
Tyr-DArg-Gly-(NMe)Phe-$NH_2$;
Tyr-DArg-Phe-Sar-ol;
Tyr-DAla-Phe-Sar-ol;
Tyr-DAla-Phe-Gly-Tyr-$NH_2$;
Gly-Tyr-DArg-Phe-Gly-$NH_2$;
Tyr-DThr-Gly-Phe-Thz-$NH_2$;
Gly-Tyr-DAla-Phe-Gly-$NH_2$;
Tyr-DAla-Phe-Gly-ol;
Tyr-DAla-Gly-(NMe)Phe-Gly-ol;
Tyr-DArg-Phe-Sar-$NH_2$;
Tyr-DAla-Phe-Sar-$NH_2$;
Tyr-Ala-Phe-Sar;
Tyr-DAla-Gly-(NMe)Phe-$NH_2$;
Sar-Tyr-DArg-Phe-Sar-$NH_2$;
Tyr-DCys-Phe-Gly-DCys-$NH_2$ (cyclisches Disulfid);
Tyr-DCys-Phe-Gly-DCys-$NH_2$ (freies Dithiol);
Tyr-DCys-Gly-Phe-DCys-$NH_2$ (cyclisches Disulfid);
Tyr-DCys-Gly-Phe-DCys-$NH_2$ (freies Dithiol);
Tyr-DAla-Phe-Gly-Tyr-Pro-Ser-$NH_2$;
Tyr-DAla-Phe-Sar-Tyr-Pro-Ser-$NH_2$;
Tyr-DAla-Phe-Sar-Phe-Pro-Ser-$NH_2$;
Tyr-DAla-Phe-Gly-Tyr-Hyp-Ser-$NH_2$;
Tyr-DAla-Phe-Sar-Tyr-Hyp-Ser-$NH_2$;
Tyr-DAla-Phe-Sar-Phe-Hyp-Ser-$NH_2$;
Tyr-DArg-Phe-Gly-Tyr-Hyp-Ser-$NH_2$;
Tyr-DArg-Phe-Sar-Tyr-Pro-Ser-$NH_2$;
Tyr-DArg-Phe-Sar-Tyr-Hyp-Ser-$NH_2$;
Tyr-DArg-Phe-Gly-Tyr-Pro-Ser-$NH_2$;
und
organischen oder anorganischen Additionssalzen jedes der Polypeptide der Gruppe 3.

**15.** Verfahren nach Anspruch 14, wobei die Polypeptide der Gruppe 1 ausgewählt sind aus jedem der Polypeptide:

(a) mit den folgenden Aminosäuresequenzen an Position 1-44 (numeriert vom terminalen N zum terminalen C).

(#144) YADAIFTNSYRKVLGQLSARKLLQDIMSRQQGESNQERGARARL-X,
(#145) YADAIFTNSYRKVLGQLSARKLLQDIMSRQQGERNQEQGARVRL-X,

(#146) YADAIFTNSYRKVLGQLSARKLLQDIMNRQQGERNQEQGAKVRL-X,
(#148) YADAIFTNSYRKILGQLSARKLLQDIMNRQQGERNQEQGAKVRL-X,
(#149) HADAIFTSSYRRILGQLYARKLLHEIMNRQQGERNQEQRSRFN-X; und funktionelle Äquivalente davon;

wobei die C-terminale Aminosäure die folgende allgemeine Rumpfformel hat:

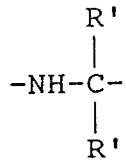

wobei jedes R' unabhängig die Substituenten des speziellen Aminosäurerestes, z.B. Wasserstoff-, Alkyl-, Aryl-, Amino- oder Säuresubstituenten darstellt; X die C-terminale Endgruppe bezeichnet und ausgewählt ist aus $-CONH_2$, -COOH, -COOR, -CONRR, $-CH_2OH$ und $-CH_2OR$, wobei R eine Alkylgruppe mit 1-6 Kohlenstoffatomen oder ein aromatischer Ring mit bis zu 12 Kohlenstoffatomen ist; und wobei die verwendeten Aminosäurerestabkürzungen mit der Standardpeptidnomenklatur übereinstimmen:

| | |
|---|---|
| G | = Gly (Glycin) |
| Y | = Tyr (L-Tyrosin) |
| I | = Ile (L-Isoleucin) |
| E | = Glu (L-Glutaminsäure) |
| T | = Thr (L-Threonin) |
| F | = Phe (L-Phenylalanin) |
| A | = Ala (L-Alanin) |
| K | = Lys (L-Lysin) |
| D | = Asp (L-Asparaginsäure) |
| C | = Cys (L-Cystein) |
| R | = Arg (L-Arginin) |
| Q | = Gln (L-Glutamin) |
| P | = Pro (L-Prolin) |
| L | = Leu (L-Leucin) |
| M | = Met (L-Methionin) |
| S | = Ser (L-Serin) |
| N | = Asn (L-Asparagin) |
| H | = His (L-Histidin) |
| W | = Trp (L-Trytophan) |
| V | = Val (L-Valin) |
| Aib | = $\alpha$-Aminobuttersäure |
| Nle | = Norleucin |
| (NMe)DAla | = N-Metyhl-D-Alanin |

(b) jedem einzelnen der Polypeptide aus (a), das die folgenden Aminosäuresubstitutionen hat:
Position 1 von (#144-#148) ist DTyr oder His;
Position 1 von (#149) ist Tyr oder DHis;
Position 2 von (#144-#149) ist (NMe)DAla oder Aib oder DAla;
Position 3 von (#144-#149) ist DAsp;
Position 4 von (#144-#149) ist DAla; und
Position 1 + 2 von (#144-#149) ist:
$DTyr^1 + DAla^2$, $DTyr^1$ + $(NMe)DAla^2$, oder $DTyr^1 + Aib^2$;
(c) jedem einzelnen der Polypeptide nach (a) oder (b), das eine Substitution von Nle für Met an Position 27 hat;
(d) jedem einzelnen der Polypeptide nach (a), (b) oder (c), bei dem die N-terminale $-NH_2$-Gruppe durch -NHCOR ersetzt ist und wobei R eine Alkylgruppe mit 1 bis 6 Kohlenstoffatomen oder ein aromatischer Ring mit bis zu 12 Kohlenstoffatomen ist;
(e) Fragmenten jedes einzelnen der Polypeptide nach (a), (b), (c) oder (d), die wenigsten die Aminosäurereste der Positionen 1-29 enthalten;

(f) mit den folgenden bestimmten Aminosäuresequenzen an Positionen 1-29 (numeriert vom terminalen N zum terminalen C):

YADAIFTNSYRKVLQQLAARKLLQDIMSR-X,
YADAIFTNSYRKVLQQLLARKLLQDIMSR-X,
YSDAIFSNAYRKILQQLLARKLLQDIMOR-X,
YADAIFSNAYRKILQQLLARKLLQDIMQR-X,
YADAIFSSAYRRLLAQLASRRLLQELLAR-X,
YADAIFTNCYRKVLCQLSARKLLQDIMSR-X (lineares Dithiol), und
YADAIFTNCYRKVLCQLSARKLLQDIMSR-X (cyclisches Disulfid);

wobei die C-terminale Aminosäure und X wie vorstehend definiert sind; und Modifikationen jeder einzelnen dieser Verbindungen nach Gruppe (f) in Übereinstimmung mit den vorstehend unter (b), (c) und (d) dargelegten Modifikationen; und

(g) organischen oder anorganischen Additionssalzen jedes der Polypeptide der Gruppe 1 nach (a), (b), (c), (d), (e) oder (f).

**16.** Verfahren nach Anspruch 14, wobei die Zusammensetzung eine Verbindung aus jeweils Polypeptiden der Gruppe 1 und Polypeptiden der Gruppe 2 enthält.

**17.** Verfahren nach Anspruch 14, wobei die Zusammensetzung eine Verbindung aus jeweils Polypeptiden der Gruppe 2 und Polypeptiden der Gruppe 3 enthält.

**18.** Verfahren nach Anspruch 14, wobei die Zusammensetzung eine Verbindung aus jeweils Polypeptiden der Gruppe 1, Polypeptiden der Gruppe 2 und Polypeptiden der Gruppe 3 enthält.

**19.** Pharmazeutische Präparation, die eine wirksame Menge der Verbindung nach einem der Ansprüche 1 bis 3 und einen inerten Trägerstoff enthält.

**20.** Pharmazeutische Präparation, die eine wirksame Menge der Zusammensetzung nach einem der Ansprüche 9 bis 13 und einen inerten Trägerstoff enthält.

## Revendications

**1.** Polypeptide capable de provoquer la libération et l'augmentation de quantités d'hormone de croissance dans le sang d'un animal receveur, ledit polypeptide étant choisi dans le groupe consistant en polypeptides définis par la structure générale :

AA1-AA2-AA3-Trp-AA5-AA6-AA7-Z,

dans laquelle AA1 est choisi dans le groupe consistant en His et 3(NMe)His (c'est-à-dire dans lequel le noyau imidazole est méthylé en position 3) ; AAO-His et AAO-3(NMe)His ; dans lequel AAO est choisi dans le groupe consistant en n'importe quel amino-acide naturel, Met(O), DOPA et Abu ;

AA2 est choisi dans le groupe consistant en DPhe, DTrp, 5-fluoro-D- ou D/LTrp ; 6-fluoro-D- ou D/LTrp (c'est-à-dire dans lequel le noyau indole est fluoré en position 5 ou 6), (formyl)DTrp (c'est-à-dire DTrp qui est formylé au niveau de l'atome d'azote du noyau indole), *XTrp, ledit *XTrp étant choisi dans le groupe consistant en les isomères de DTrp N-monométhylés (c'est-à-dire $N^{\alpha}$Me)DTrp et (indole NMe)DTrp), $D^{\alpha}$Nal et $D^{\beta}$Nal ;

AA3 est choisi dans le groupe consistant en Ala, Gly et Ser ;

AA5 est choisi dans le groupe consistant en DPhe et (NMe)DPhe ;

AA6 est choisi dans le groupe consistant en tous les L-amino-acides naturels, des dipeptides des L-amino-acides naturels, par exemple Ala-Ala, et des composés de formule :

$H_2N-(CH_2)_n-CO_2H$,

dans laquelle n = 2-12 ;

AA7 est choisi dans le groupe consistant en Arg, iLys, Lys et Orn ;

Z représente le groupe à l'extrémité C-terminale dudit polypeptide ou le ou les amino-acides C-terminaux plus le groupe terminal, Z étant choisi dans le groupe consistant en $-CONH_2$, -COOH, -COOR, -CONHR, $-CONR_2$, $-CH_2OH$ et $-CH_2OR$, dans lesquels R représente un groupe alkyle ayant 1 à 6 atomes de carbone ou un noyau aromatique ayant jusqu'à 12 atomes de carbone ; Z étant choisi, en variante, dans le groupe consistant en -Gly-Z', -Met-Z', -Lys-Z', -Cys-Z', -Gly-Tyr-Z' et -Ala-Tyr-Z',

dans lesquels Z' est choisi dans le groupe consistant en $-CONH_2$, -COOH, -CONHR, -COOR, $CONR_2$, $-CH_2OH$ et $-CH_2OR$, dans lesquels R répond à la définition précitée ;

et les sels d'addition organiques ou inorganiques de n'importe lesquels desdits polypeptides ;

les abréviations des résidus d'amino-acides utilisées étant conformes à la nomenclature usuelle des peptides :

| | | |
|---|---|---|
| Gly | = | glycine |
| Tyr | = | L-tyrosine |
| Ile | = | L-isoleucine |
| Glu | = | Acide L-glutamique |
| Thr | = | L-thréonine |
| Phe | = | L-phénylalanine |
| Ala | = | L-alanine |
| Lys | = | L-lysine |
| Asp | = | Acide L-aspartique |
| Cys | = | L-cystéine |
| Arg | = | L-arginine |
| Gln | = | L-glutamine |
| Pro | = | L-proline |
| Leu | = | L-leucine |
| Met | = | L-méthionine |
| Ser | = | L-sérine |
| Asn | = | L-asparagine |
| His | = | L-histidine |
| Trp | = | L-tryptophane |
| Val | = | L-valine |
| DOPA | = | 3,4-dihydroxyphénylalanine |
| Met(O) | = | Méthionine-sulfoxyde |
| Abu | = | Acide $\alpha$-aminobutyrique |
| iLys | = | $N^{\epsilon}$-isopropyl-L-lysine |
| 4-Abu | = | Acide 4-aminobutyrique |
| Orn | = | L-ornithine |
| $D^{\alpha}$Nal | = | $\alpha$-naphtyl-D-alanine |
| $D^{\beta}$Nal | = | $\beta$-naphtyl-D-alanine |

Toutes les abréviations d'amino-acides en trois lettres précédées par une lettre "D" indiquent la configuration D du résidu d'amino-acide ; les abréviations précédées par "D/L" indiquent un mélange des configurations D et L des amino-acides désignés ; et la glycine entre dans le cadre de la définition de l'expression "L-amino-acides naturels".

**2.** Polypeptide suivant la revendication 1, qui est choisi dans le groupe consistant en :

AA1-AA2-AA3-Trp-AA5-Ala-AA7-$NH_2$ ;

AA1-AA2-AA3-Trp-AA5-Ala-Ala-AA7-$NH_2$ ;

et

des sels d'addition organiques ou inorganiques de n'importe lesquels desdits polypeptides.

**3.** Polypeptide suivant la revendication 1, qui est choisi dans le groupe consistant :

His-DTrp-Ala-Trp-DPhe-Ala-Lys-$NH_2$ ;

His-DTrp-Ala-Trp-DPhe-Ala-Ala-Lys-$NH_2$ ; et

des sels d'addition organiques ou inorganiques de l'un et l'autre desdits polypeptides.

**4.** Procédé pour provoquer la libération et l'élévation de taux sanguins d'hormone de croissance chez des animaux par administration à ces animaux d'une quantité efficace d'au moins un des polypeptides définis dans l'une quelconque des revendications 1, 2 et 3, à l'extrusion de n'importe quelle méthode de traitement thérapeutique de l'organisme de l'homme ou d'un animal.

**5.** Composé de formule :

AA1-AA2-AA3-Trp-AA5-AA6-AA7-Ⓡ,

dans laquelle AA1 est choisi dans le groupe consistant en His et 3(NMe)His (c'est-à-dire dans lequel le noyau imidazole est méthylé en position 3) ; AAO-His, AAO-3(NMe)His, dans lequel AAO est n'importe quel amino-acide naturel ;

AA2 est choisi dans le groupe consistant en DPhe, DTrp, 5-fluoro-D- ou D/LTrp ; 6-fluoro-D- ou D/LTrp (c'est-à-dire dans lequel le noyau indole est fluoré en position 5 ou 6), (formyl)DTrp (c'est-à-dire DTrp qui est formylé au niveau de l'atome d'azote du noyau indole), *XTrp, ledit *XTrp étant choisi dans le groupe consistant en les isomères de DTrp N-monométhylés (c'est-à-dire $N^{\alpha}$Me)DTrp et (indole NMe)DTrp), $D^{\alpha}$Nal et $D^{\beta}$Nal ; AA3 est choisi dans le groupe consistant en Ala,

Gly et Ser ;

AA5 est choisi dans le groupe consistant en DPhe et (NMe)DPhe ;

AA6 est choisi dans le groupe consistant en tous les L-amino-acides naturels, des dipeptides des L-amino-acides naturels, par exemple Ala-Ala, et des composés de formule :

$H_2N-(CH_2)_n-CO_2H$,

dans laquelle n = 2-12 ;

AA7 est choisi dans le groupe consistant en Arg, iLys, Lys et Orn ;

et dans laquelle Ⓡ représente une résine polymérique, et les groupes fonctionnels des amino-acides constitutifs sont protégés avec des groupes protecteurs convenables, de la manière requise.

6. Composé de formule :

His-DTrp-Ala-Trp-DPhe-Ala-Lys-Ⓡ;

dans laquelle Ⓡ représente une résine polymérique, et les groupes fonctionnels des amino-acides constitutifs sont protégés avec des groupes protecteurs convenables, de la manière requise.

7. Composé de formule :

His-DTrp-Ala-Trp-DPhe-Ala-Ala-Lys-Ⓡ;

dans laquelle Ⓡ représente une résine polymérique, et les groupes fonctionnels des amino-acides constitutifs sont protégés avec des groupes protecteurs convenables, de la manière requise.

8. Polypeptide lié à une résine, choisi dans le groupe consistant en :

DTrp-Ala-Trp-DPhe-Ala-Ala-Lys-Ⓡ,
DTrp-Ala-Trp-DPhe-Ala-Lys-Ⓡ,
Ala-Trp-DPhe-Ala-Ala-Lys-Ⓡ,
Ala-Trp-DPhe-Ala-Lys-Ⓡ,
Trp-DPhe-Ala-Ala-Lys-Ⓡ,
Trp-DPhe-Ala-Lys-Ⓡ,
Dphe-Ala-Ala-Lys-Ⓡ et
DPhe-Ala-Lys-Ⓡ.

dans lequels R représente une résine polymérique, et les groupes fonctionnels des amino-acides constitutifs sont protégés avec des groupes protecteurs convenables, de la manière requise.

9. Association efficace pour provoquer la libération et l'élévation de la quantité d'hormone de croissance dans le sang d'un animal, l'association comprenant une quantité efficace de polypeptides choisis parmi au moins deux groupes différents des polypeptides du Groupe 1, des polypeptides du Groupe 2 et des polypeptides du Groupe 3 ;

dans laquelle les polypeptides du Groupe 1 sont choisis parmi n'importe lesquelles des hormones naturelles de libération de l'hormone de croissance et leurs équivalents fonctionnels, lesdits polypeptides agissant au niveau du récepteur de l'hormone de libération d'hormone de croissance de mammifères et d'autres vertébrés, ainsi que de crustacés ;

les polypeptides du Groupe 2 sont choisis parmi n'importe lesquels des polypeptides ayant la structure :

AA1-AA2-AA3-Trp-AA5-AA6-AA7-Z,

dans laquelle AA1 est choisi dans le groupe consistant en His et 3(NMe)His (c'est-à-dire dans lequel le

noyau imidazole est méthylé en position 3) ; AAO-His et AAO-3(NMe)His ; dans lequel AAO représente n'importe quel amino-acide naturel,

AA2 est choisi dans le groupe consistant en DPhe, DTrp, 5-fluoro-D- ou D/LTrp ; 6-fluoro-D- ou D/LTrp (c'est-à-dire dans lequel le noyau indole est fluoré en position 5 ou 6), (formyl)DTrp (c'est-à-dire DTrp qui est formylé au niveau de l'atome d'azote du noyau indole), *XTrp, ledit *XTrp étant choisi dans le groupe consistant en les isomères de DTrp N-monométhylés (c'est-à-dire $N^{\alpha}$Me)DTrp et (indole NMe)DTrp), $D^{\alpha}$Nal et $D^{\beta}$Nal ;

AA3 est choisi dans le groupe consistant en Ala, Gly et Ser ;

AA5 est choisi dans le groupe consistant en DPhe et (NMe)DPhe ;

AA6 est choisi dans le groupe consistant en tous les L-amino-acides naturels, des dipeptides des L-amino-acides naturels, par exemple Ala-Ala, et des composés de formule :

$H_2N-(CH_2)_n-CO_2H$,

dans laquelle n = 2-12 ;

AA7 est choisi dans le groupe consistant en Arg, iLys, iLys et Orn ;

Z représente le groupe à l'extrémité C-terminale dudit polypeptide ou le ou les amino-acides C-terminaux plus le groupe terminal, Z étant choisi dans le groupe consistant en $-CONH_2$, -COOH, -COOR, -CONHR, $-CONR_2$, $-CH_2OH$ et $-CH_2OR$, dans lesquels R représente un groupe alkyle ayant 1 à 6 atomes de carbone ou un noyau aromatique ayant jusqu'à 12 atomes de carbone ; et Z est choisi, en variante, dans le groupe consistant en -Gly-Z', -Met-Z', -Lys-Z', -Cys-Z', -Gly-Tyr-Z' et -Ala-Tyr-Z', dans lesquels Z' est choisi dans le groupe consistant en $-CONH_2$, -COOH, -CONHR, -COOR, $-CONR_2$, $-CH_2OH$ et $-CH_2OR$, dans lesquels R répond à la définition précitée ;

et des sels d'addition organiques ou inorganiques de n'importe lesquels desdits polypeptides ;

les abréviations des résidus d'amino-acides utilisées étant conformes à la nomenclature usuelle des peptides :

| | | |
|---|---|---|
| Gly | = | glycine |
| Tyr | = | L-tyrosine |
| Ile | = | L-isoleucine |
| Glu | = | Acide L-glutamique |
| Thr | = | L-thréonine |
| Phe | = | L-phénylalanine |
| Ala | = | L-alanine |
| Lys | = | L-lysine |
| Asp | = | Acide L-aspartique |
| Cys | = | L-cystéine |
| Arg | = | L-arginine |
| Gln | = | L-glutamine |
| Pro | = | L-proline |
| Leu | = | L-leucine |
| Met | = | L-méthionine |
| Ser | = | L-sérine |
| Asn | = | L-asparagine |
| His | = | L-histidine |
| Trp | = | L-tryptophane |
| Val | = | L-valine |
| DOPA | = | 3,4-dihydroxyphénylalanine |
| Gly-ol | = | 2-aminoéthanol |
| Hyp | = | Trans-4-hydroxy-L-proline |
| Met(O) | = | Méthionine-sulfoxyde |
| Met(O)-ol | = | Méthionine-sulfoxyde-alcool |
| Sar | = | Sarcosine |
| Sar-ol | = | Sarcosine-alcool |
| Thz | = | Acide L-thiazolidine-4-carboxylique |
| iLys | = | $N^{\epsilon}$-isopropyl-L-lysine |
| 4-Abu | = | Acide 4-aminobutyrique |
| Orn | = | L-ornithine |
| $D^{\alpha}$nal | = | $\alpha$-naphtyl-D-alanine |

$D^{\beta}$Nal = $\beta$-naphtyl-D-alanine

Toutes les abréviations d'amino-acides en trois lettres précédées par un "D" indiquent la configuration D du résidu d'amino-acide ; les abréviations précédées par "D/L" indiquent un mélange des configurations D et L des amino-acides désignés ; et la glycine entre dans la définition de l'expression "L-amino-acides naturels" ; et

les polypeptides du groupe 3 sont choisis parmi n'importe lesquels des polypeptides ayant la structure :

Tyr-DArg-Phe-$NH_2$ ;
Tyr-DAla-Phe-$NH_2$ ;
Tyr-DArg($NO_2$)-Phe-$NH_2$ ;
Tyr-DMet(O)-Phe-$NH_2$;
Tyr-DAla-Phe-Gly-$NH_2$;
Tyr-DArg-Phe-Gly-$NH_2$;
Tyr-DThr-Phe-Gly-$NH_2$;
Phe-DArg-Phe-Gly-$NH_2$;
Tyr-DArg-Phe-Sar;
Tyr-DAla-Gly-Phe-$NH_2$;
Tyr-DArg-Gly-Trp-$NH_2$;
Tyr-DArg-($NO_2$)-Phe-Gly-$NH_2$;
Tyr-DMet(O)-Phe-Gly-$NH_2$;
(NMe)Tyr-DArg-Phe-Sar-$NH_2$;
Tyr-DArg-Phe-Gly-ol;
Tyr-DArg-Gly-(NMe)Phe-$NH_2$;
Tyr-DArg-Phe-Sar-ol;
Tyr-DAla-Phe-Sar-ol;
Tyr-DAla-Phe-Gly-Tyr-$NH_2$;
Gly-Tyr-DArg-Phe-Gly-$NH_2$;
Tyr-DThr-Gly-Phe-Thz-$NH_2$;
Gly-Tyr-DAla-Phe-Gly-$NH_2$;
Tyr-DAla-Phe-Gly-ol;
Tyr-DAla-Gly-(NMe)Phe-Gly-ol;
Tyr-DArg-Phe-Sar-$NH_2$;
Tyr-DAla-Phe-Sar-$NH_2$;
Tyr-DAla-Phe-Sar;
Tyr-DAla-Gly-(NMe)Phe-$NH_2$;
Sar-Tyr-DArg-Phe-Sar-$NH_2$;
Tyr-DCys-Phe-Gly-DCys-$NH_2$ (disulfure cyclique)
Tyr-DCys-Phe-Gly-DCys-$NH_2$ (dithiol libre)
Tyr-DCys-Gly-Phe-DCys-$NH_2$ (disulfure cyclique)
Tyr-DCys-Gly-Phe-DCys-$NH_2$ (dithiol libre)
Tyr-DAla-Phe-Gly-Tyr-Pro-Ser-$NH_2$;
Tyr-DAla-Phe-Sar-Tyr-Pro-Ser-$NH_2$;
et

des sels d'addition organiques et inorganiques de n'importe lesquels desdits polypeptides du Groupe 3 ;

ladite association étant administrée en un rapport tel que ladite association soit efficace pour provoquer la libération synergique et l'élévation de la quantité d'hormone de croissance dans le sang d'un tel animal.

**10.** Association suivant la revendication 9, dans laquelle les polypeptides du Groupe 1 sont choisis parmi :

(a) n'importe lesquels des polypeptides ayant les séquences d'amino-acides suivantes en les positions 1-44 (numérotées de l'extrémité N-terminale à l'extrémité C-terminale) :

(N° 144) YADAIFTNSYRKVLGQLSARKLLQDIMSRQQGESNQERGARARL-X,
(N° 145) YADAIFTNSYRKVLGQLSARKLLQDIMSRQQGERNQEQGARVRL-X,
(N° 146) YADAIFTNSYRKVLGQLSARKLLQDIMNRQQGERNQEQGAKVRL-X,
(N° 148) YADAIFTNSYRKILGQLSARKLLQDIMNRQQGERNQEQGAKVRL-X,
(N° 149) HADAIFTSSYRRILGQLYARKLLHEIMNRQQGERNQEQRSRFN-X ;

et leurs équivalents fonctionnels ;

dans lesquelles l'amino-acide C-terminal répond à la formule générale tronquée suivante :

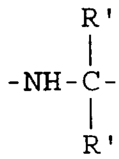

dans laquelle chaque groupe R' représente indépendamment les substituants du résidu d'amino-acide particulier, par exemple l'hydrogène, des substituants alkyle, aryle, amino ou acide ; X représente le groupe à l'extrémité C-terminale et est choisi entre $-CONH_2$, -COOH, -COOR, -CONRR, $-CH_2OH$ et $-CH_2OR$, dans lesquels R représente un groupe alkyle ayant 1 à 6 atomes de carbone ou un noyau aromatique ayant jusqu'à 12 atomes de carbone ; et les abréviations des résidus d'amino-acides utilisées étant conformes à la nomenclature usuelle des peptides :

G = Gly (glycine),
Y = Tyr (L-tyrosine),
I = Ile (L-isoleucine),
E = Glu (acide L-glutamique),
T = Thr (L-thréonine),
F = Phe (L-phénylalanine),
A = Ala (L-alanine),
K = Lys (L-lysine),
D = Asp (Acide L-aspartique),
C = Cys (L-cystéine),
R = Arg (L-arginine),
Q = Gln (L-glutamine),
P = Pro (L-proline),
L = Leu (L-leucine),
M = Met (L-méthionine),
S = Ser (L-sérine),
N = Asn (L-asparagine),
H = His (L-histidine),
W = Trp (L-tryptophane) et
V = Val (L-valine) ;
Nle = Norleucine
Sar = Sarcosine
Sar-ol = Sarcosine-alcool
Gly-ol = 2-amino-éthanol
Met(O) = Méthionine-sulfoxyde

(b) n'importe lesquels des polypeptides (a) ayant les substitutions d'amino-acides suivantes :
la position 1 du (n° 144-148) est occupée par DTyr ou His ;
la position 1 du (n° 149) est occupée par Tyr ou DHis ;
la position 2 du (n° 144-149) est occupée par (NMe)DAla ou Aib ou bien DAla ;
la position 3 du (n° 144-149) est occupée par DAsp ;
la position 4 du (n° 144-149) est occupée par DAla ; et
la position 1 + 2 du (n° 144-149) est occupée par :
$DTyr^1$ + $DAla^2$, $DTyr^1$ + $(NMe)DAla^2$, ou $DTyr^1$ = $Aib^2$ ;

(c) n'importe lequel desdits polypeptides (a) ou (b) ayant une substitution de Met par Nle en position 27 ;

(d) n'importe lequel desdits polypeptides (a), (b) ou (c) dans lequel le groupe $-NH_2$ N-terminal est remplacé par un groupe -NHCOR dans lequel R représente un groupe alkyle ayant 1 à 6 atomes de carbone ou un noyau aromatique ayant jusqu'à 12 atomes de carbone ;

(e) des fragments de n'importe lequel des polypeptides (a), (b), (c) ou (d), qui contiennent au moins les résidus d'amino-acides des positions 1-29 ;

(f) n'importe lesquels des polypeptides ayant les séquences d'amino-acides spécifiques suivantes en positions 1-29 (numérotées de l'extrémité N-terminale à l'extrémité C-terminale) :

YADAIFTNSYRKVLQQLAARKLLQDIMSR-X,

YADAIFTNSYRKVLQQLLARKLLQDIMSR-X,
YSDAIFSNAYRKILQQLLARKLLQDIMOR-X,
YADAIFSNAYRKILQQLLARKLLQDIMQR-X,
YADAIFSSAYRRLLAQLASRRLLQELLAR-X,
YADAIFTNCYRKVLCQLSARKLLQDIMSR-X (dithiol linéaire) et
YADAIFTNCYRKVLCQLSARKLLQDIMSR-X (disulfure cyclique) ;

dans lesquelles l'amino-acide C-terminal et X répondent aux définitions précitées ; et une modification de n'importe lequel de ces composés du groupe (f) conformément aux modifications indiquées ci-dessus en (b), (c) et (d) ; et

(g) des sels d'addition organiques ou inorganiques de n'importe lesquels desdits polypeptides (a), (b), (c), (d), (e) ou (f) du Groupe 1.

**11.** Association suivant la revendication 9, comprenant un composé choisi parmi les polypeptides du Groupe 1 et un composé choisi parmi les polypeptides du Groupe 2.

**12.** Association suivant la revendication 9, comprenant un composé choisi parmi les polypeptides du Groupe 2 et un composé choisi parmi les polypeptides du Groupe 3.

**13.** Association suivant la revendication 9, comprenant un composé choisi parmi les polypeptides du Groupe 1, un composé choisi parmi les polypeptides du Groupe 2 et un composé choisi parmi les polypeptides du Groupe 3.

**14.** Méthode pour provoquer la libération et l'élévation de la quantité d'hormone de croissance dans le sang d'un animal ; à l'exclusion de n'importe quelle méthode de traitement thérapeutique de l'organisme de l'homme ou d'un animal, comprenant l'administration d'une dose efficace d'une association comprenant des polypeptides choisis parmi au moins deux groupes différents des polypeptides du Groupe 1, des polypeptides du Groupe 2 et des polypeptides du Groupe 3 ;

dans laquelle les polypeptides du Groupe 1 sont choisis parmi n'importe lesquels des hormones naturelles de libération de l'hormone de croissance et de leurs équivalents fonctionnels, lesdits polypeptides agissant au niveau du récepteur d'hormone de libération de l'hormone de croissance de mammifères et d'autres vertébrés, ainsi que de crustacés ;

les polypeptides du Groupe 2 sont choisis parmi n'importe lesquels des polypeptides ayant la structure :

AA1-AA2-AA3-Trp-AA5-AA6-AA7-Z,

dans laquelle AA1 est choisi dans le groupe consistant en His et 3(NMe)His (c'est-à-dire dans lequel le noyau imidazole est méthylé en position 3) ; AAO-His et AAO-3(NMe)His ; dans lequel AAO représente n'importe quel amino-acide naturel ;

AA2 est choisi dans le groupe consistant en DPhe, DTrp, 5-fluoro-D- ou D/LTrp ; 6-fluoro-D- ou D/LTrp (c'est-à-dire dans lequel le noyau indole est fluoré en position 5 ou 6), (formyl)DTrp (c'est-à-dire un DTrp qui est formylé au niveau de l'atome d'azote du noyau indole), *XTrp, ledit *XTrp étant choisi dans le groupe consistant en les isomères de DTrp N-monométhylés (c'est-à-dire $N^\alpha$Me)DTrp et (indole NMe)DTrp), $D^\alpha$Nal et $D^\beta$Nal ;

AA3 est choisi dans le groupe consistant en Ala, Gly et Ser ;

AA5 est choisi dans le groupe consistant en DPhe et (NMe)DPhe ;

AA6 est choisi dans le groupe consistant en tous les L-amino-acides naturels, des dipeptides des L-amino-acides naturels, par exemple Ala-Ala, et des composés de formule :

$H_2N-(CH_2)_n-CO_2H$,

dans laquelle n = 2-12 ;

AA7 est choisi dans le groupe consistant en Arg, iLys, Lys et Orn ;

Z représente le groupe à l'extrémité C-terminale dudit polypeptide ou le ou les amino-acides C-terminaux plus le groupe terminal, Z étant choisi dans le groupe consistant en $-CONH_2$, -COOH, -COOR, -CONHR, $-CONR_2$, $-CH_2OH$ et $-CH_2OR$, dans lesquels R représente un groupe alkyle ayant 1 à 6 atomes de carbone ou un noyau aromatique ayant jusqu'à 12 atomes de carbone ; et Z, en variante, étant choisi dans le groupe consistant en -Gly-Z', -Met-Z', -Lys-Z', -Cys-Z', -Gly-Tyr-Z' et -Ala-Tyr-Z', dans lesquels Z' est choisi dans le groupe consistant en $-CONH_2$, -COOH, -CONHR, -COOR, $-CONR_2$, $-CH_2OH$ et $-CH_2OR$, dans lesquels R répond à la définition précitée ;

et les sels d'addition organiques ou inorganiques de n'importe lesquels desdits polypeptides ;

les abréviations des résidus d'amino-acides utilisées étant conformes à la nomenclature usuelle des peptides :

Gly = glycine
Tyr = L-tyrosine
Ile = L-isoleucine
Glu = acide L-glutamique
Thr = L-thréonine
Phe = L-phénylalanine
Ala = L-alanine
Lys = L-lysine
Asp = Acide L-aspartique
Cys = L-cystéine
Arg = L-arginine
Gln = L-glutamine
Pro = L-proline
Leu = L-leucine
Met = L-méthionine
Ser = L-sérine
Asn = L-asparagine
His = L-histidine
Trp = L-tryptophane
Val = L-valine
iLys = $N^{\epsilon}$-isopropyl-L-lysine
4-Abu = Acide 4-aminobutyrique
Nle = Norleucine
Sar = Sarcosine
Sar-ol = Sarcosine-alcool
Gly-ol = 2-amino-éthanol
Met(O) = Méthionine-sulfoxyde
Orn = L-ornithine
$D^{\alpha}$nal = $\alpha$-naphtyl-D-alanine
$D^{\beta}$Nal = $\beta$-naphtyl-D-alanine

Toutes les abréviations d'amino-acides en trois lettres précédées par une lettre "D" indiquant la configuration D du résidu d'amino-acide ;

les polypeptides du Groupe 3 sont choisis parmi n'importe lesquels des polypeptides ayant la structure :

Tyr-DArg-Phe-$NH_2$;
Tyr-DAla-Phe-$NH_2$;
Tyr-DArg($NO_2$)-Phe-$NH_2$;
Tyr-DMet(O)-Phe-$NH_2$;
Tyr-DAla-Phe-Gly-$NH_2$;
Tyr-DArg-Phe-Gly-$NH_2$;
Tyr-DThr-Phe-Gly-$NH_2$;
Phe-DArg-Phe-Gly-$NH_2$;
Tyr-DArg-Phe-Sar;
Tyr-DAla-Gly-Phe-$NH_2$;
Tyr-DArg-Gly-Trp-$NH_2$;
Tyr-DArg($NO_2$)-Phe-Gly-$NH_2$;
Tyr-DMet(O)-Phe-Gly-$NH_2$;
(NMe)Tyr-DArg-Phe-Sar-$NH_2$;
Tyr-DArg-Phe-Gly-ol;
Tyr-DArg-Gly-(NMe)Phe-$NH_2$;
Tyr-DArg-Phe-Sar-ol;
Tyr-DAla-Phe-Sar-ol;
Tyr-DAla-Phe-Gly-Tyr-$NH_2$;
Gly-Tyr-DArg-Phe-Gly-$NH_2$;
Tyr-DThr-Gly-Phe-Thz-$NH_2$;

Gly-Tyr-DAla-Phe-Gly-$NH_2$;
Tyr-DAla-Phe-Gly-ol;
Tyr-DAla-Gly-(NMe)Phe-Gly-ol;
Tyr-DArg-Phe-Sar-$NH_2$;
Tyr-DAla-Phe-Sar-$NH_2$;
Tyr-DAla-Phe-Sar;
Tyr-DAla-Gly-(NMe)Phe-$NH_2$;
Sar-Tyr-DArg-Phe-Sar-$NH_2$;
Tyr-DCys-Phe-Gly-DCys-$NH_2$ (disulfure cyclique)
Tyr-DCys-Phe-Gly-DCys-$NH_2$ (dithiol libre)
Tyr-DCys-Gly-Phe-DCys-$NH_2$ (disulfure cyclique)
Tyr-DCys-Gly-Phe-DCys-$NH_2$ (dithiol libre)
Tyr-DAla-Phe-Gly-Tyr-Pro-Ser-$NH_2$;
Tyr-DAla-Phe-Sar-Tyr-Pro-Ser-$NH_2$;
Tyr-DAla-Phe-Sar-Phe-Pro-Ser-$NH_2$;
Tyr-DAla-Phe-Gly-Tyr-Hyp-Ser-$NH_2$;
Tyr-DAla-Phe-Sar-Tyr-Hyp-Ser-$NH_2$;
Tyr-DAla-Phe-Sar-Phe-Hyp-Ser-$NH_2$;
Tyr-DArg-Phe-Gly-Tyr-Hyp-Ser-$NH_2$;
Tyr-DArg-Phe-Sar-Tyr-Pro-Ser-$NH_2$;
Tyr-DArg-Phe-Sar-Tyr-Hyp-Ser-$NH_2$;
Tyr-DArg-Phe-Gly-Tyr-Pro-Ser-$NH_2$;
et des sels d'addition organiques et inorganiques de n'importe lesquels desdits polypeptides du Groupe 3.

**15.** Méthode suivant la revendication 14, dans lequel les polypeptides du Groupe 1 sont choisis parmi :
(a) n'importe lesquels des polypeptides ayant les séquences d'amino-acides suivantes en positions 1-44 (numérotées de l'extrémité N-terminale à l'extrémité C-terminale) :
(N° 144) YADAIFTNSYRKVLGQLSARKLLQDIMSRQQGESNQERGARARL-X,
(N° 145) YADAIFTNSYRKVLGQLSARKLLQDIMSRQQGERNQEQGARVRL-X,
(N° 146) YADAIFTNSYRKVLGQLSARKLLQDIMNRQQGERNQEQGAKVRL-X,
(N° 148) YADAIFTNSYRKILGQLSARKLLQDIMNRQQGERNQEQGAKVRL-X,
(N° 149) HADAIFTSSYRRILGQLYARKLLHEIMNRQQGERNQEQRSRFN-X;
et leurs équivalents fonctionnels ;
dans lesquelles l'amino-acide C-terminal répond à la formule générale tronquée suivante :

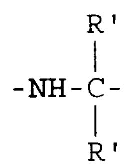

dans laquelle chaque groupe R' représente indépendamment les substituants du résidu d'amino-acide particulier, par exemple l'hydrogène, des substituants alkyle, aryle, amino ou acide ; X représente le groupe à l'extrémité C-terminale et est choisi entre -$CONH_2$, -COOH, -COOR, -CONRR, -$CH_2OH$ et -$CH_2OR$, dans lesquels R représente un groupe alkyle ayant 1 à 6 atomes de carbone ou un noyau aromatique ayant jusqu'à 12 atomes de carbone ; et dans lesquelles les abréviations des résidus d'amino-acides utilisées sont conformes à la nomenclature usuelle des peptides :

| | |
|---|---|
| G | = Gly (glycine), |
| Y | = Tyr (L-tyrosine), |
| I | = Ile (L-isoleucine), |
| E | = Glu (acide L-glutamique), |
| T | = Thr (L-thréonine), |
| F | = Phe (L-phénylalanine), |
| A | = Ala (L-alanine), |
| K | = Lys (L-lysine), |

D = Asp (Acide L-aspartique),
C = Cys (L-cystéine),
R = Arg (L-arginine),
Q = Gln (L-glutamine),
P = Pro (L-proline),
L = Leu (L-leucine),
M = Met (L-méthionine),
S = Ser (L-sérine),
N = Asn (L-asparagine),
H = His (L-histidine),
W = Trp (L-tryptophane) et
V = Val (L-valine) ;
Aib = acide $\alpha$-aminoisobutyrique
Nle = Norleucine
(NMe)DAla = N-méthyl-D-alanine

(b) n'importe lesquels desdits polypeptides (a) ayant les substitutions d'amino-acides suivantes :
la position 1 du (n° 144 - n° 148) est occupée par DTyr ou His ;
la position 1 du (n° 149) est occupée par Tyr ou DHis ;
la position 2 du (n° 144 - n° 149) est occupée par (NMe)DAla ou Aib ou bien DAla ;
la position 3 du (n° 144 - n° 149) est occupée par DAsp ;
la position 4 du (n° 144 - n° 149) est occupée par DAla ; et
la position 1 + 2 du (n° 144 - n° 149) est occupée par :
$DTyr^1 + DAla^2$, $DTyr^1 + (NMe)DAla^2$, ou $DTyr^1 = Aib^2$ ;

(c) n'importe lequel desdits polypeptides (a) ou (b) ayant une substitution de Met par Nle en position 27 ;

(d) n'importe lequel des polypeptides (a), (b) ou (c) dans lequel le groupe $-NH_2$ N-terminal est remplacé par un groupe -NHCOR dans lequel R représente un groupe alkyle ayant 1 à 6 atomes de carbone, ou un noyau aromatique ayant jusqu'à 12 atomes de carbone ;

(e) des fragments de n'importe lequel desdits polypeptides (a), (b), (c) ou (d), qui contiennent au moins les résidus d'amino-acides en positions 1-29 ;

(f) n'importe lesquels des polypeptides ayant les séquences d'amino-acides spécifiques suivantes en positions 1-29 (numérotées de l'extrémité N-terminale à l'extrémité C-terminale) :
YADAIFTNSYRKVLQQLAARKLLQDIMSR-X,
YADAIFTNSYRKVLQQLLARKLLQDIMSR-X,
YSDAIFSNAYRKILQQLLARKLLQDIMOR-X,
YADAIFSNAYRKILQQLLARKLLQDIMQR-X,
YADAIFSSAYRRLLAQLASRRLLQELLAR-X,
YADAIFTNCYRKVLCQLSARKLLQDIMSR-X (dithiol linéaire) et
YADAIFTNCYRKVLCQLSARKLLQDIMSR-X (disulfure cyclique) ;
dans lesquelles l'amino-acide C-terminal et X répondent aux définitions précitées ; et une modification de n'importe lequel de ces composés du groupe (f) conformément aux modifications indiquées ci-dessus en (b), (c) et (d) ; et

(g) des sels d'addition organiques ou inorganiques de n'importe lequel des polypeptides (a), (b), (c), (d), (e) ou (f) du Groupe 1.

**16.** Méthode suivant la revendication 14, dans laquelle l'association comprend un composé choisi parmi les polypeptides du Groupe 1 et un composé choisi parmi les polypeptides du Groupe 2.

**17.** Méthode suivant la revendication 14, dans laquelle l'association comprend un composé choisi parmi les polypeptides du Groupe 2 et un composé choisi parmi les polypeptides du Groupe 3.

**18.** Méthode suivant la revendication 14, dans laquelle l'association comprend un composé choisi parmi les polypeptides du Groupe 1, un composé choisi parmi les polypeptides du Groupe 2 et un composé choisi parmi les polypeptides du Groupe 3.

**19.** Préparation pharmaceutique qui comprend une quantité efficace du composé suivant l'une quelconque des revendications 1 à 3 et un support inerte.

**20.** Préparation pharmaceutique qui comprend une quantité efficace de l'association suivant l'une quelconque des revendications 9 à 13 et un support inerte.